(19) Europäisches Patentamt
European Patent Office
Office européen des brevets

(11) EP 3 289 976 A1

(12) EUROPEAN PATENT APPLICATION

(43) Date of publication:
07.03.2018 Bulletin 2018/10

(51) Int Cl.:
A61B 5/145 (2006.01)          A61B 5/00 (2006.01)
A61B 5/1495 (2006.01)

(21) Application number: 17188116.2

(22) Date of filing: 28.08.2017

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(30) Priority: 31.08.2016  JP 2016170372

(71) Applicant: SYSMEX CORPORATION
Kobe-shi
Hyogo 651-0073 (JP)
(72) Inventors:
• Hori, Nobuyasu
  Hyogo, 651-0073 (JP)
• Suminaka, Chihiro
  Hyogo, 651-0073 (JP)
• Kojima, Junko
  Hyogo, 651-0073 (JP)

(74) Representative: Hoffmann Eitle
Patent- und Rechtsanwälte PartmbB
Arabellastraße 30
81925 München (DE)

(54) **SENSOR ASSEMBLY, TEST SUBSTANCE MONITORING SYSTEM, AND TEST SUBSTANCE MONITORING METHOD**

(57)     Disclosed are a novel means capable of accurately calculating the amount of in-vivo components as a test substance, and its uses.

A sensor assembly 20 includes an electrochemical sensor 30 for measuring a test substance percutaneously extracted from a living body, and a processor 115a for calculating the intensity of the background signal at the measurement time point of the test substance after the elapse of the predetermined time based on the change over time of the intensity of the background signal measured within a predetermined time before measurement of the test substance by the electrochemical sensor 30. In this way, the amount of the in-vivo component as the test substance can be accurately calculated since the calculated value of the intensity of the background signal to be subtracted from the unprocessed signal including the signal attributable to the test substance can be obtained.

FIG. 1

EP 3 289 976 A1

**Description**

FIELD OF THE INVENTION

The invention relates to a sensor assembly, test substance monitoring system, and test substance monitoring method.

BACKGROUND

An in-vivo component contained in a specimen such as blood, for example, glucose, is an indicator of diabetes. The in-vivo component must be detected with high accuracy in order to manage the amount of in-vivo component for preventing disease deterioration.

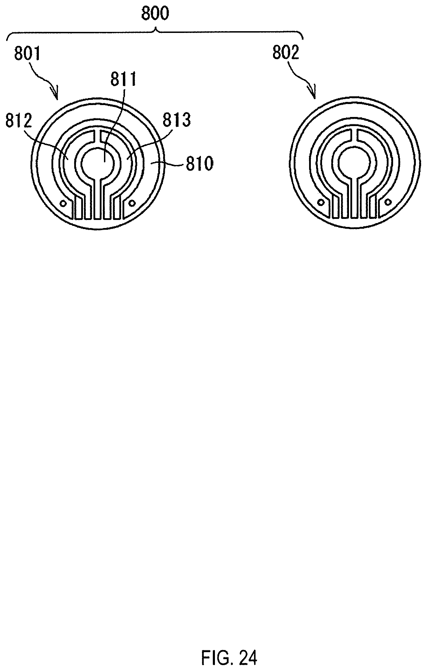

Japanese Patent Registration No. 4545398 discloses a monitoring device that measures the amount of glucose contained in a specimen collected percutaneously. As shown in FIG. 24, the monitoring device 800 described in Japanese Patent Registration No. 4545398 includes two detection devices 801 and 802 attached to the subject's arm. The detection devices 801 and 802 each include a working electrode 811, a reference electrode 812, and a counter electrode 813. Glucose is extracted from the subject's skin by one detection device 801 and the extracted glucose is measured. The signal measurement value obtained by the detection device 801 includes a signal originating from glucose and a background signal that is not attributable to glucose. Therefore, only the background signal is measured by the other detection device 802 simultaneously with the measurement of glucose by the detection device 801, and the signal value due to glucose is calculated by subtracting the background signal measurement value acquired by the detection device 802 from the signal measurement value acquired by the detection device 801. The amount of glucose is acquired based on the calculated signal measurement value.

SUMMARY OF THE INVENTION

However, since the background signal is measured by different detection devices in the monitoring device 800 described in Japanese Patent Registration No. 4545398, variations may occur in the background signal measurement value between the detection device 801 and the detection device 802 due to the individual difference of the detection devices. Therefore, the background signal measurement value obtained by the detection device 802 may be different from the background signal measurement value that originally should be subtracted from the detection device 801 in the monitoring device 800 described in Japanese Patent Registration No. 4545398. In this case, it was difficult to accurately calculate the amount of glucose.

A sensor assembly according to a first aspect of the present invention includes an electrochemical sensor for measuring a test substance percutaneously extracted from a living body, and a processor for determining the intensity of the background signal at an optional measurement time point of the test substance outside a predetermined time based on the intensity of the background signal measured within the predetermined time by the electrochemical sensor.

The electrochemical sensor is used for applications in which a test substance is detected by a change in the intensity of an electric signal. A "background signal" is a signal detected regardless of the presence or absence of a test substance, and refers to a signal generated by a sensor assembly without originating from a test substance. A "background signal" also includes a signal attributable to the electrochemical sensor itself. For example, when a hydrogel is used as a collecting member for collecting a test substance and an enzyme sensor is used as an electrochemical sensor, the "background signal" includes a current signal inherent to the electrochemical sensor, a current signal flowing when a component in the hydrogel diffuses on the electrode, a current signal flowing when a component in the hydrogel reacts on the electrode, and a current signal flowing when a component fixed on the electrode reacts. The "predetermined time" may be any time other than the time during which the measurement of the test substance is performed. The "predetermined time" may be, for example, the time just before or immediately after the measurement of the test substance or may be a time when manufacturing the sensor assembly.

The background signal is usually different for each sensor assembly. In the sensor assembly according to the first aspect of the invention, the electrochemical sensor used for the measurement of the test substance is also used for obtaining the background signal alone, and calculates the intensity of the background signal at the measurement time point of the test substance outside the predetermined time based on the intensity of the background signal at the time of measurement of the test substance within the predetermined time. According to the sensor assembly having such a configuration, therefore, it is possible to determine the intensity of the background signal to be originally subtracted from the intensity of the signal obtained by measuring the test substance since the intensity of the background signal at the time of measurement of the test substance is determined based on the background signal obtained from the electrochemical sensor used for measurement of the test substance. Therefore, it is possible to accurately calculate the amount of the test substance according to the sensor assembly of the first aspect of the present invention.

The predetermined time may be a period of time from the energizing of the electrochemical sensor to the stabilization of the value measured by the electrochemical sensor. Normally, when an electrochemical sensor is used, after a voltage is applied to the electrochemical sensor, a certain amount of time is required for the current signal flowing in the electrochemical sensor to stabilize and for the value measured by the electrochemical sensor to stabilize after a voltage is applied to the electrochemical sensor. When a test substance is measured using an electrochemical sensor, the measurement value also tends to vary during the period from the application of a voltage to the electrochemical sensor until the measured value by the electrochemical sensor becomes stabilized. However, the present inventors measured the intensity of the background signal during the time from the application of the voltage to the electrochemical sensor until the value measured by the electrochemical sensor became stabilized, and based on the measurement result, it was found that the amount of the test substance can be accurately calculated using this time by determining the intensity of the background signal at the time of measurement.

The electrochemical sensor is configured to measure a test substance extracted from a living body to a collection member while in a state of contact with a collection member, and the electrochemical sensor also may measure the intensity of the background signal within a predetermined time while in a state of contact with the collection member, the predetermined time being a time before the test substance is extracted to the collecting member after the electrochemical sensor is brought into contact with the collecting member. In this way, the intensity of the background signal to be subtracted as previously described can be more accurately determined since the intensity of the background signal caused by the collection member also can be measured, and the intensity of the background signal at the time of measurement of the test substance can be determined based on this measurement value.

The processor also may calculate the intensity of the background signal at the time of measurement based on the change over time in the intensity of the background signal measured within a predetermined time. In this way, the intensity of the background signal at the time of measurement of the test substance can be calculated, taking into consideration the temporal fluctuation of the intensity of the background signal.

The processor preferably calculates the intensity of the background signal at the measurement time point by applying exponential approximation, exponential approximation, linear approximation or a combination thereof to the temporal change of the intensity of the background signal within a predetermined time. The processor also preferably calculates the intensity of the background signal at the measurement time point based on an equation obtained by calculating constants included in the equation by performing power approximation, exponential approximation, linear approximation or performing fitting of combinations thereof with respect to the temporal change of the intensity of the background signal within the predetermined time. The temporal change in the intensity of the background signal is a power function change, an exponential change, a linear change or a combination thereof. Therefore, the calculated value of the intensity of the background signal can be obtained with higher accuracy according to the sensor assembly having such a configuration.

The processor is configured to obtain a value representing the intensity of the signal attributable to the test substance by subtracting the value of the intensity of the background signal at the measurement time point from the measurement value of the intensity of the unprocessed signal including the signal attributable to the test substance acquired by the electrochemical sensor at the time of measurement of the test substance. "Signal attributable to a test substance" means a signal that varies according to the amount of the test substance. "Unprocessed signal including a signal attributable to a test substance" includes a signal attributable to a test substance and a background signal.

The electrochemical sensor also may be configured to automatically obtain a value representing the intensity of the signal attributable to the test substance by measuring the intensity of the unprocessed signal including the signal attributable to the test substance and subtracting the intensity of the background signal at the measurement time point when the measurement of the intensity of the background signal and the determination of the intensity of the background signal at the measurement time point are automatically performed in a first measurement performed after the sensor assembly is first activated, and second and subsequent measurements are performed. In this way, the burden on the user can be reduced since the user does not need to manually switch between the background measurement and the measurement of the test substance.

The processor can obtain a value indicating the amount of the test substance based on the intensity of the signal attributable to the test substance and the in-vivo concentration of a substance correlated with the in-vivo concentration of the test substance. The in-vivo concentration of the substance correlated with the in-vivo concentration of the test substance may be, for example, the blood concentration of the test substance, the concentration of other substances correlated with the test substance in the interstitial fluid, the blood concentration of other substances correlated with the test substance in the blood and the like, but the correlated substance is not particularly limited. The in-vivo concentration of the substance correlated with the in-vivo concentration of the test substance is preferably the blood concentration of the test substance. According to the sensor assembly having such a configuration, it is possible to accurately estimate the amount of the test substance.

The sensor assembly may be equipped with one electrochemical sensor.

The sensor assembly further preferably includes a memory for storing the value of the intensity of the background signal at the time of measurement. In this case, the user reduce the time required to prepare for measurement because the intensity of the unprocessed signal including the signal attributable to the test substance can be measured without measuring the intensity of the background signal since the intensity can be measured by using the value of the intensity of the background signal at the measurement time point stored in the memory.

The electrochemical sensor can measure a test substance at each of a plurality of measurement time points outside the predetermined time. In this case, the processor can determine the intensity of the background signal at each of the plurality of measurement time points based on the intensity of the background signal measured within a predetermined time.

The electrochemical sensor also may be an enzyme sensor provided with a substrate body, an electrode disposed on the substrate body, and an enzyme immobilized on the surface of the electrode. According to the sensor assembly having such a configuration, it is possible to accurately calculate the amount of a test substance using an enzymatic reaction.

The test substance also may be glucose. In this case, the enzyme sensor is a glucose sensor including a substrate body, an electrode disposed on the substrate body for detecting hydrogen peroxide, and glucose oxidase immobilized on the surface of the electrode. According to the sensor assembly having such a configuration, the amount of glucose can be calculated with high accuracy.

A monitoring system according to a second aspect of the invention includes a collection member capable of collecting a test substance contained in interstitial fluid extracted from a living body, and the aforementioned sensor assembly.

The collection member also may include hydrogel. In this case, the predetermined time may be a time for the diffusion of the substance in the hydrogel to proceed to achieve a state in which the influence on the measurement of the test substance reaches equilibrium. state

The test substance monitoring method according to a third aspect of the invention is a method of percutaneously extracting and monitoring a test substance from a living body. The test substance monitoring method of the third aspect of the invention includes (A1) a step of bringing a collection member into contact with an electrochemical sensor and measuring the intensity of the background signal within a predetermined time; (A2) a step of determining the intensity of the background signal at an optional measurement time point of the test substance outside the predetermined time based on the intensity of the background signal within the predetermined time; (A3) a step of measuring a test substance extracted into a collection member from a living body by the electrochemical sensor at the measurement time point to obtain a measurement value of the intensity of the unprocessed signal including the signal attributable to the test substance; and (A4) a step of subtracting the value of the intensity of the background signal obtained in the step (A2) from the measured value of the intensity of the unprocessed signal measured in the step (A3) to calculate the intensity of the signal attributable to the test substance. According to the monitoring method of the test substance adopting such an operation, the amount of the test substance can be calculated with high accuracy.

In step (A1), an electrochemical sensor is brought into contact with a collecting member attached to the living body, and the intensity of the background signal can be measured within a predetermined time from when the electrochemical sensor in contact with the collecting member is energized to when the measured value by the electrochemical sensor stabilizes.

In step (A1), the intensity of the background signal can be measured within a predetermined time after the electrochemical sensor is brought into contact with the collecting member and before the test substance is collected by the collecting member.

In step (A2), the intensity of the background signal at each of a plurality of measurement time points of the test substance is determined based on the intensity of the background signal within the predetermined time. In this case, in step (A3), a test substance is measured at each of the plurality of measurement time points, and a measurement value of the intensity of a unprocessed signal including the signal attributable to the test substance is acquired at each measurement time point. In the step (A4), the value of the intensity of the background signal can be subtracted from the measured value of the intensity of the unprocessed signal acquired at each of the plurality of measurement points.

Disclosed are a novel means capable of accurately calculating the amount of in-vivo component as a test substance, and its uses.

BRIEF DESCRIPTION OF THE DRAWINGS

FIG. 1 is a brief explanatory diagram showing a continuous monitoring system;
FIG. 2 is a schematic exploded view showing a sensor assembly;
FIG. 3 is a schematic perspective view showing a sensor assembly;
FIG. 4 is a block diagram showing a functional configuration of a continuous monitoring system;

FIG. 5 is a schematic plane view showing a glucose sensor;

FIG. 6 is a schematic cross sectional view showing a hydrogen peroxide electrode of the glucose sensor;

FIG. 7 is a schematic cross sectional view showing a collection member;

FIG. 8 is a flowchart showing a processing procedure by a continuous monitoring system;

FIG. 9 is a schematic cross sectional view showing skin having micropores formed therein;

FIG. 10 is a schematic cross sectional view showing a collection member mounted on the skin;

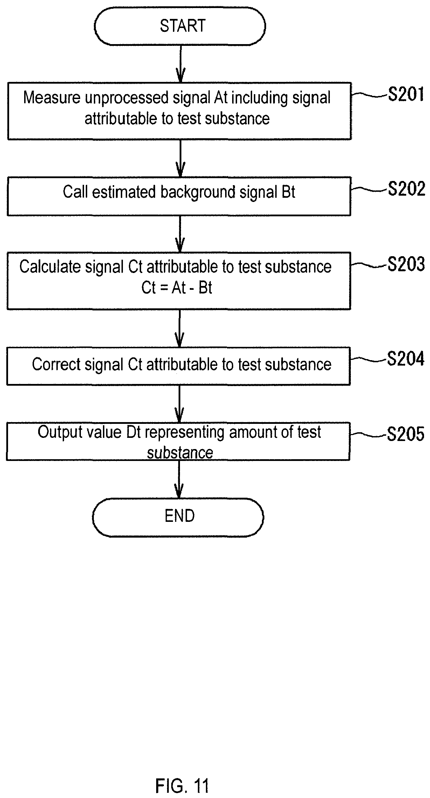

FIG. 11 is a flowchart showing a processing procedure by a continuous monitoring system;

FIG. 12 is a graph showing the results of investigating changes over time in blood glucose level in Example 1

FIG. 13 is a graph showing a Clarke error grid;

FIG. 14 is a graph showing a result of evaluating an estimated blood glucose level by error grid analysis in Example 1;

FIG. 15 is a graph showing a result of investigating the change over time in blood glucose level in Comparative Example 1;

FIG. 16 is a graph showing a result of evaluating an estimated blood glucose level by error grid analysis in Comparative Example 1;

FIG. 17 is a graph showing the results of investigating changes over time in blood glucose level in Example 2;

FIG. 18 is a graph showing a result of evaluating an estimated blood glucose level by error grid analysis in Example 2;

FIG. 19 is a graph showing a result of investigating the change over time in blood glucose level in Comparative Example 2;

FIG. 20 is a graph showing a result of evaluating an estimated blood glucose level by error grid analysis in Comparative Example 2;

FIG. 21 is a graph showing the results of investigating changes over time in blood glucose level in Example 3;

FIG. 22 is a graph showing a result of evaluating an estimated blood glucose level by error grid analysis in Example 3;

FIG. 23 is a graph showing a result of measurement of a background current signal and calculation of an estimated background current signal; and

FIG. 24 is a schematic explanatory view showing a monitoring device described in Japanese Patent Registration No. 4545398.

## DESCRIPTION OF THE EMBODIMENTS OF THE INVENTION

Description of Terms

In this specification, the description of numerical ranges by endpoints such as "X to Y" includes all numbers and rational numbers included in each range and endpoints described.

"Microinvasive interstitial fluid extraction technique" refers to a technique of extracting interstitial fluid from a subject with minimal invasion. In microinvasive interstitial fluid extraction technology, micropores are formed in the skin of the living body by a puncture tool or the like to promote extraction of interstitial fluid. The interstitial fluid extracted from the micropores is collected using a collection member including a gel or the like.

"In-vivo component" refers to a component contained in a living body, for example, a body fluid extracted from a subject. Examples of in-vivo components include, but are not limited to, glucose, uric acid, lactic acid, galactose and the like. "Test substance" refers to a substance to be detected among in-vivo components. Examples of body fluid include, but are not limited to, interstitial fluid, blood and the like.

Continuous Monitoring System

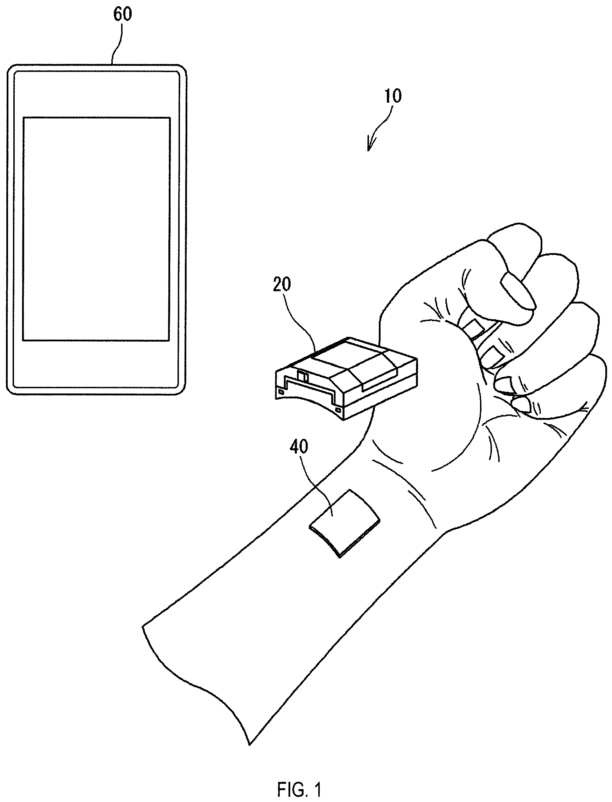

Hereinafter, a continuous monitoring system will be described with reference to the drawings. As shown in FIG. 1, the continuous monitoring system 10 includes a sensor assembly 20, a collection member 40, and a terminal 60. The continuous monitoring system 10 is used by mounting the sensor assembly 20 so as to contact the collection member 40 installed on the forearm of the subject who has formed micropores. Terminal 60 is used to display the information obtained by sensor assembly 20. For example, a cellular phone terminal, a tablet type computer and the like can be mentioned as the terminal 60, but there is no particular limitation.

Sensor Assembly

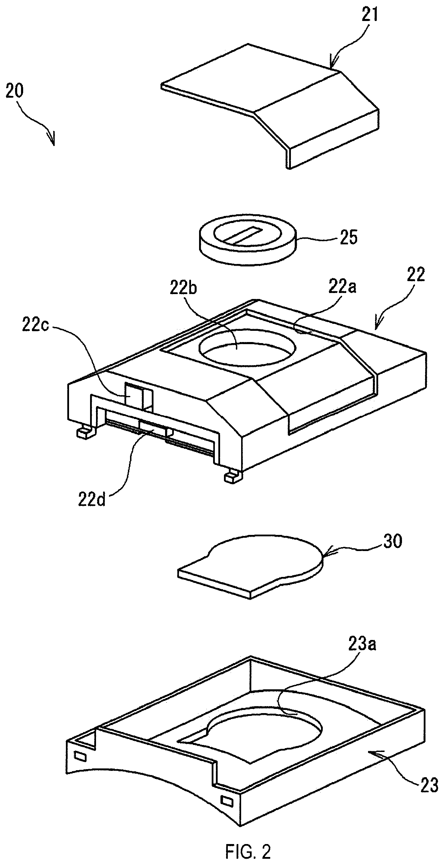

As shown in FIG. 2, the sensor assembly 20 includes a cover 21, an upper main body 22 incorporating an electric circuit, and a lower main body 23 for housing the electrochemical sensor 30. The upper body 22 includes an electric circuit (not shown), a notch part 22a for attaching the cover 21, a housing 22b for housing the battery 25 as a power supply, a switch 22c for switching on/off of the sensor assembly 20, and a connection terminal 22d for connecting the electrochemical sensor 30 to the electric circuit. The cover 21 is attached to the notch part 22a so as to cover the housing 22b in which the battery 25 is stored. The electric circuit includes a connection terminal to the battery 25, a processor, a memory and the like. The lower main body 23 has a hole 23a for aligning the electrode-side surface of the electrochemical sensor 30 with the micropore-formed area of the subject's forearm.

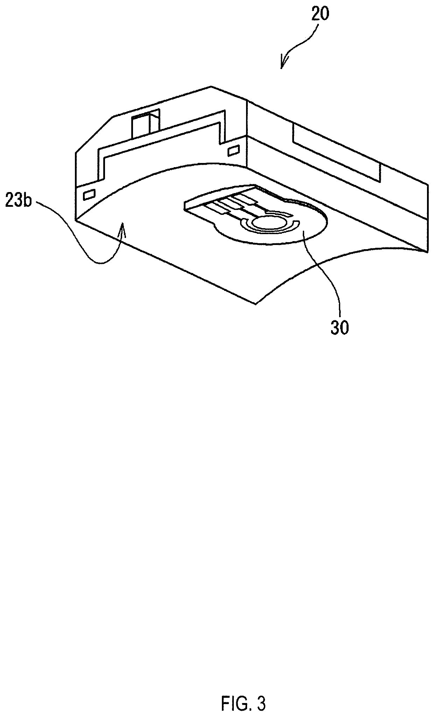

As shown in FIG. 3, the forearm contact surface 23b of the lower body 23 of the sensor assembly 20 is a recess having a curved surface along the curved surface of the forearm so that the sensor assembly 20 can be attached to the forearm. The R value of the contact surface 23b is normally set to 40 to 50.

Returning to FIG. 2, the electrochemical sensor 30 is connected to the electric circuit via the connection terminal 22d of the upper main body 22. The electrochemical sensor 30 connected to the connection terminal 22d also is housed between the upper main body 22 and the lower main body 23. At this time, as shown in FIG. 3, the electrochemical sensor 30 is housed between the upper main body 22 and the lower main body 23 so that the electrodes are positioned on the forearm side.

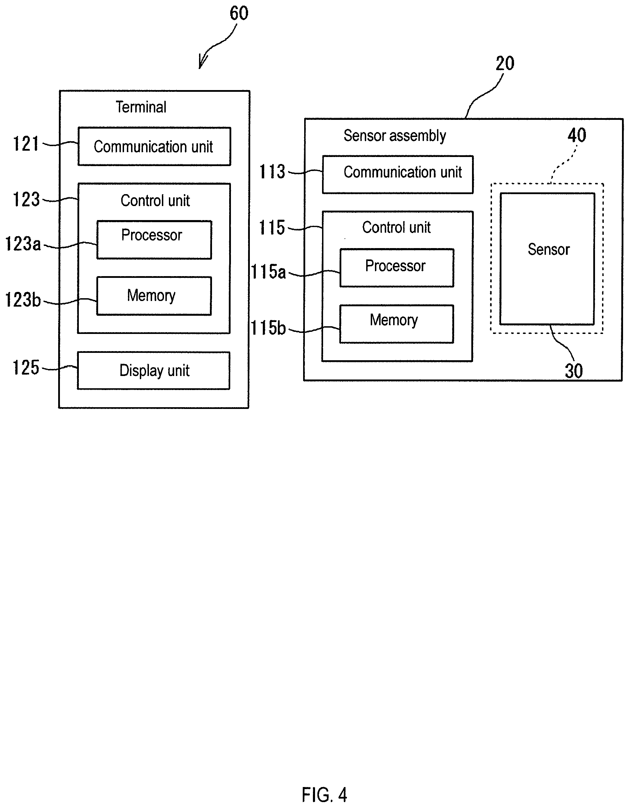

As shown in FIG. 4, the sensor assembly 20 has an electrochemical sensor 30, a communication unit 113, and a control unit 115. The sensor assembly 20 is communicably connected to the communication unit 121 of the terminal 60 via the communication unit 113.

The electrochemical sensor 30 is disposed so as to be in contact with the collection member 40. The measurement value of the intensity of the unprocessed signal and the measurement value of the background signal including the signal attributable to the test substance acquired by the electrochemical sensor 30 are output to the control unit 115.

In the present embodiment, the communication unit 113 is a wireless communication unit. Note that the communication unit 113 also may be a wired communication unit. Since the handling by the user is easy, the communication unit 113 is preferably a wireless communication unit.

The control unit 115 of the sensor assembly 20 includes a processor 115a and a memory 115b.

The processor 115a has the function of acquiring information on measured values of intensities of unprocessed signals and information on measured values of background signals including signals attributable to the test substance output from the electrochemical sensor 30. The processor 115a stores the information of the measurement value of the intensity of the unprocessed signal including the signal attributable to the acquired test substance and the information of the measurement value of the background signal in the memory 115b as necessary. The processor 115a has a function of executing the calculation of the intensity of the background signal at an arbitrary point in time after elapse of a predetermined time applying a power approximate expression, an exponential approximate expression, a linear approximate expression, or a combination thereof as the estimated expression for the change over time of the intensity of the background signal acquired within a predetermined time by the electrochemical sensor 30. The processor 115a also stores the information of the calculated value of the intensity of the background signal in the memory 115b as necessary. Furthermore, the processor 115a has a function of executing the calculation of the intensity of the signal attributable to the test substance by subtracting the intensity of the background signal at the measurement time point of the intensity of the unprocessed signal including the signal attributable to the test substance from the measured value of the intensity of the unprocessed signal including the signal attributable to the test substance. The processor 115a also stores the information on the intensity of the signal attributable to the test substance in the memory 115b as necessary. The processor 115a also has a function of executing the calculation of the amount of the test substance based on the relationship between the intensity of the signal attributable to the test substance and the in-vivo concentration of a substance correlated with the in-vivo concentration of the test substance. Further, the processor 115a stores the information of the amount of the test substance in the memory 115b as necessary. Note that, the start of the predetermined time may be, for example, a point of time when the power supply of the sensor assembly 20 is turned on, a point of several minutes after the point of turning on the power of the sensor assembly 20, a point of time instructed by the user and the like. The end of the predetermined time also includes, for example, the time when the user instructs termination, the time when the background signal becomes lower than a threshold value, a time set in the memory 115b of the sensor assembly 20 set in advance or the like.

The memory 115b stores information on a power approximate expression, an exponential approximation expression, a linear approximation expression or a combination thereof which is an estimated expression, information on the in-vivo concentration of a substance correlated with the in-vivo concentration of the test substance, information on correction formula and the like. Note that, examples of the in-vivo concentration of the substance correlated with the in vivo concentration of the test substance include, but are not limited to, the blood concentration of the test substance, the concentration in the interstitial fluid of another substance correlated with the test substance, blood concentration of other substances correlated with the test substance and the like. The memory 115b also may temporarily store information on the measured value of the intensity of the background signal acquired within a predetermined time by the electrochemical sensor 20, information on the calculated value of the intensity of the background signal at an optional point in time after the lapse of a predetermined time, information on the intensity of a signal attributable to the test substance, information on the amount of a test substance and the like.

Note that the sensor assembly 20 also may be integrated with the collection member 40 described later. In this case, measurement of the background signal is performed without setting the sensor assembly 20 on the arm of the subject.

Electrochemical Sensor Structure

In the following description, examples of an electrochemical sensor are described, including a glucose sensor for use in detection of glucose contained in interstitial fluid extracted from a subject.

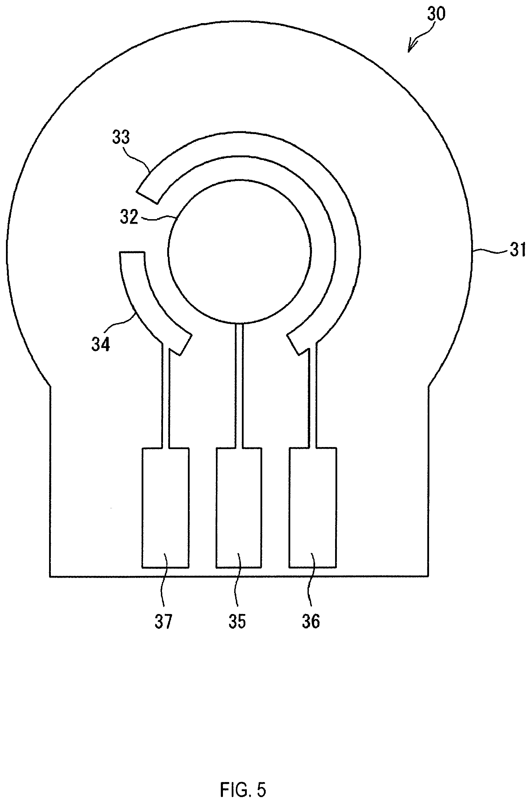

As shown in FIG. 5, the electrochemical sensor 30 includes a substrate 31, a working electrode 32, a counter electrode 33, a reference electrode 34, and electrode leads 35, 36, and 37. The working electrode 32, the counter electrode 33, the reference electrode 34, and the electrode leads 35, 36, 37 are provided on one surface of the substrate 31.

The shape of the substrate 31 may be a rectangular shape, a polygonal shape, a circular shape, or the like, and is not particularly limited. The material configuring the substrate 31 is an insulating material which does not affect the conductivity of at least the working electrode 32, the counter electrode 33 and the reference electrode 34. Examples of the material configuring the substrate 31 include polyester resins such as polyvinyl alcohol, polyethylene terephthalate, polybutylene terephthalate, polyethylene naphthalate, and polybutylene naphthalate; polyimide; glass epoxy resin; glass; ceramic and the like, but is not particularly limited. The thickness of the substrate 31 can be appropriately decided according to the application of the electrochemical sensor and the like. The substrate 31 preferably is sufficiently flexible to be adhered to the skin.

As shown in FIG. 5, the working electrode 32 is arranged on one side of the surface of the substrate 31. The working electrode 32 is connected to the electrode lead 35. The electrode lead 35 extends from the working electrode 32 toward the other side portion of the substrate 31, more specifically, toward the lower side of the substrate 31 in FIG. 5.

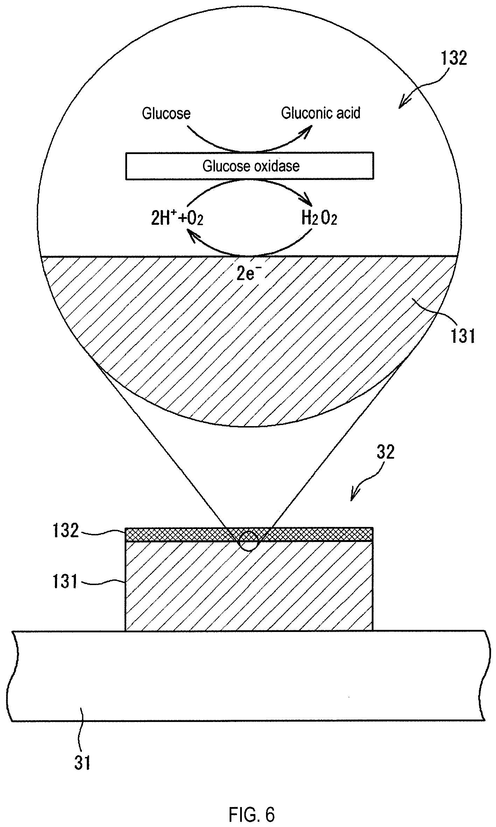

As shown in FIG. 6, the working electrode 32 includes an electrode layer 131 formed on one surface of the substrate 31 and an enzyme layer 132 containing glucose oxidase. The enzyme layer 132 is provided on the surface of the electrode layer 131.

The electrode layer 132 exchanges electrons with hydrogen peroxide attributable to glucose. The electrode layer 131 contains a conductive material. A metal material such as platinum, gold and the like can be used as the conductive material used for the electrode layer 131, but there is no particular limitation. The thickness of the electrode layer 132 is not particularly limited as long as it is a thickness that maintains conductivity. From the perspective of conductivity, the thickness of the electrode layer 132 is preferably 0.01 $\mu$m or more, more preferably 0.05 $\mu$m or more, and from the perspective of manufacturing cost the thickness is preferably 0.5 $\mu$m or less, more preferably 0 .3 $\mu$m or less.

The enzyme layer 132 is provided on the surface of the electrode layer 131. In the embodiment, the enzyme layer 132 includes glucose oxidase. Glucose oxidase is an enzyme that generates hydrogen peroxide using glucose as a substrate. The organism of the source of glucose oxidase is not particularly limited. The amount of glucose oxidase in the enzyme layer 132 is preferably 0.01 U/$\mu$L or more, and more preferably 0.1 U/$\mu$L or more from the viewpoint of glucose detection sensitivity when converted to the amount of glucose oxidase activity in the solution applied on the electrode layer 131. From the perspective of cost, the amount of glucose oxidase is preferably 100 U/$\mu$L or less, and more preferably 50 U/$\mu$L or less.

The working electrode 32 is brought into contact with body fluid. When glucose is contained in the body fluid, the glucose oxidase existing in the enzyme layer 132 in the working electrode 32 oxidizes the glucose, as shown in FIG. 6. Oxidation of glucose produces hydrogen peroxide and gluconic acid. The generated hydrogen peroxide is electrolyzed on the electrode layer 131 of the working electrode 32. According to the electrochemical sensor 20, glucose contained in a body fluid can be detected based on a current signal generated by electrolysis of hydrogen peroxide.

Returning to FIG. 5, the counter electrode 33 is disposed outside the working electrode 32, more specifically, on the surface of the substrate 31 on the upper side of the working electrode 32 in FIG. 5. The counter electrode 32 is connected to the electrode lead 36. The electrode lead 36 extends from the counter electrode 33 toward the other side portion of the substrate 31, more specifically, toward the lower side of the substrate 31 in FIG. 5. The counter electrode 33 includes an electrode layer containing a conductive material. The conductive material may be, for example, metals such as platinum, gold, silver, copper, carbon, palladium, chromium, aluminum, nickel, alloys including at least one of these metals, metal halogens such as chlorides of these metals, carbon materials such as carbon nanotube film, glassher carbon, graphite and the like, semiconductors such as silicon and germanium, and is not particularly limited. The thickness of the electrode layer in the counter electrode 33 can be appropriately determined according to the application of the electrochemical sensor and the like.

In FIG. 5, the reference electrode 34 is disposed at a position facing the counter electrode 33 across the working electrode 32. The reference electrode 34 is connected to the electrode lead 37. The electrode lead 37 extends from the reference electrode 34 toward the other side portion of the substrate 31, more specifically, toward the lower side of the substrate 31 in FIG. 5. The reference electrode 34 includes an electrode layer containing a conductive material. The conductive material used for the electrode layer of the reference electrode 34 is the same as the conductive material used for the electrode layer of the counter electrode 33. Specific examples of the reference electrode include a silver-silver chloride electrode and the like, but are not particularly limited. The thickness of the electrode layer in the reference electrode 34 can be appropriately determined according to the application of the electrochemical sensor 30 and the like. The electrochemical sensor 30 also may omit the reference electrode 34. In this case, the counter electrode 33 also may serve as the reference electrode depending on the type of the conductive material used for the counter electrode 33, the thickness of the counter electrode 33 and the like. In the case of measuring a large current, the electrochemical sensor 30 preferably includes the reference electrode 34 from the perspective of suppressing the influence of the voltage drop and stabilizing the voltage applied to the working electrode 32.

Examples of a method of forming the working electrode 32, the counter electrode 33, and the reference electrode 34 include, for example, a vapor deposition method, a sputtering method, a screen printing method, and the like, but are not particularly limited. The method of forming the working electrode 32, the counter electrode 33, and the reference electrode 34 can be appropriately selected according to the type of the conductive material.

The electrode lead 35, the electrode lead 36, and the electrode lead 37 are arranged so as to be parallel to each other at the other side part of the substrate 31, more specifically, at the lower side portion of the substrate 31 in FIG 5.

The electrochemical sensor 30 may be an enzyme sensor other than a glucose sensor, such as a protein sensor, a DNA sensor or the like. In this case, in the electrochemical sensor 30, instead of glucose oxidase contained in the enzyme layer 132 of the working electrode 32, can also use other enzymes, substances specifically binding to the protein of the test substance, or substance specific to the DNA of the test substance. It is possible to detect substances other than glucose according to the electrochemical sensor 30 of a type other than the glucose sensor. For example, an enzyme that generates hydrogen peroxide by an enzymatic reaction typified by lactate oxidase, galactose oxidase, cholesterol oxidase and the like, an enzyme that generates a substance other than hydrogen peroxide by an enzymatic reaction such as glucose dehydrogenase may be used as the enzyme, but is not particularly limited. When an enzyme that generates a substance other than hydrogen peroxide by an enzymatic reaction is used as the enzyme, platinum, gold, silver, copper, carbon, palladium, chromium, aluminum, nickel or the like can be used as the conductive material of the working electrode, an alloy containing at least one of these metals, a metal halide such as chloride of these metals, a carbon material such as a carbon nanotube film, glassher carbon, graphite, a semiconductor such as silicon, germanium, or the like may be used. Examples of substances that specifically bind to proteins include, but are not limited to, antibodies against proteins, and the like. Examples of substances that specifically bind to DNA include, but are not limited to, complementary strand DNA of the DNA of the test substance, and the like.

The electrode structure of the electrochemical sensor 30 also may be a three-electrode structure or a two-electrode structure. The three-electrode structure is a three-electrode structure including one working electrode 32, one counter electrode 33, and one reference electrode 34 as described above. The two-electrode structure is a two-electrode structure including one working electrode, and an electrode that serves as both a counter electrode and reference electrode.

Collection Member

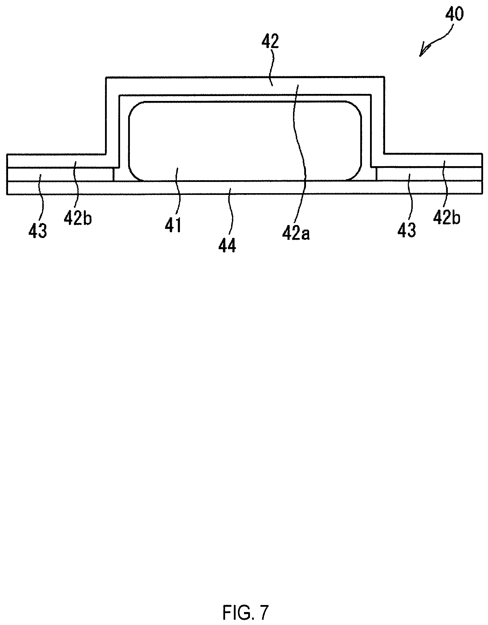

As shown in FIG. 7, the collection member 40 is a member for collecting interstitial fluid extracted from the skin of the subject by percutaneous sampling. The collection member 40 has an extraction medium 41 capable of collecting interstitial fluid and a support member 42 supporting the extraction medium. Examples of the extraction medium include, but are not limited to, hydrogel, pure water, phosphate buffered saline, tris-buffered physiological saline and the like. A hydrogel is preferable when considering the motion of the subject at the time of percutaneous sampling of the interstitial fluid. The hydrogel only needs to be capable of collecting the test substance in the interstitial fluid or interstitial fluid. Examples of hydrogel materials include, but are not limited to, agarose-based hydrogels; hydrogels based on polyethylene glycol diacrylate; hydrogels based on vinyl acetate containing polyethylene glycol/polyethyleneimine diacrylate and polyethylene glycol-n-vinylpyrrolidone diacrylate and the like. The polymer capable of forming a hydrogel may be either a synthetic polymer or a natural polymer. Examples of synthetic polymers include, but are not limited to, polyvinyl alcohol, polyacrylic acid polymers, cellulose derivatives such as hydroxypropyl cellulose, polymers such as polyethylene glycol, copolymers and block copolymers, and other water-swellable or biocompatible polymers. Examples of natural polymers include, but are not limited to, collagen, hyaluronic acid, gelatin, albumin, polysaccharides, derivatives thereof and the like. The natural polymer may be of a compound type separated from various botanical materials such as psyllium. The hydrogel can also be produced by, for example, the following methods: a) synthesis from a monomer (crosslinking polymerization); b) synthesis from a polymer and a polymerization aid (grafting and crosslinking polymerization); c) synthesis from nonpolymerization aid (crosslinking polymer); d) synthesis from polymer with energy source (crosslinking polymer without auxiliary agent), and e) synthesis from polymer (due to intermolecular bond of reactive polymer crosslinking).

The thickness of the hydrogel preferably is as thin as possible in consideration of the response speed of the electrochemical sensor 30 to the test substance. The thickness of the hydrogel is preferably 1 to 1000 $\mu$m, more preferably 5 to 700 $\mu$m, most preferably 10 to 300 $\mu$m. The size of the hydrogel may be any size as long as the interstitial fluid extraction region is covered and contact with the electrochemical sensor 30 can be secured. Note that the hydrogel may contain an enzyme when the test substance is a low molecular weight compound such as glucose, lactic acid or the like. Examples of the enzyme include glucose oxidase, glucose dehydrogenase, lactate oxidase and the like. The method of introducing the enzyme into the hydrogel can be appropriately determined depending on the method of forming the hydrogel. Examples of methods for introducing the enzyme into the hydrogel in the case of preparing a hydrogel by electron beam crosslinking of polyvinyl alcohol include, but are not limited to, a method of adding an enzyme to a polyvinyl solution and performing electron beam crosslinking, and a method in which a functional group is introduced into a polymer constituting the hydrogel and the enzyme is chemically bonded to the functional group, and the like. In this case, the working electrode of the electrochemical sensor 30 is a working electrode which does not include an enzyme layer and includes an electrode layer.

The support member 42 has a support part body 42a having a concave portion and a flange portion 42b formed on the outer peripheral side of the support part body 42a. The extraction medium is held in the concave portion of the support part body 42a. An adhesive layer 43 is provided on the surface of the flange portion 42b. In the state before use, a release paper 44 for sealing the extraction medium 41 held in the concave portion is affixed to the adhesive layer 43. When conducting the measurement, the release paper 44 is peeled from the adhesive layer 43 to expose the extraction medium 41 and the adhesive layer 43. The collection member 40 can be affixed to the skin of the subject by the adhesive layer 43 so that the extraction medium 41 is brought into contact with the skin of the subject.

Terminal

As shown in FIG. 4, the terminal 60 includes a communication unit 121, a control unit 123, and a display unit 125.
The terminal 60 is communicably connected to the communication unit 113 of the sensor assembly 20 via the communication unit 121. The communication unit 121 is a wireless communication unit. Note that the communication unit 121 also may be a wired communication unit. Since the handling by the user is easy, the communication unit 121 is preferably a wireless communication unit. Note that the terminal 60 also may calculate a value indicating the amount of the test substance or the like.
The control unit 123 includes a processor 123a and a memory 123b.
The processor 123a has the function of obtaining information on the measurement value of the intensity of an unprocessed signal including a signal attributable to the test substance, information on the measurement value of the background signal, information of the calculated value of the intensity of the background signal at an optional time point after a predetermined time has elapsed, information on the intensity of the signal attributable to the test substance, information on the amount of the test substance and the like from the sensor assembly via the communication unit 12. If necessary, the processor 123a also stores information on the measured value of the intensity of the unprocessed signal including the signal attributable to the test substance, the information on the measured value of the background signal in the absence of the test substance, information on the calculated value of the intensity of the background signal at an optional time point after a predetermined time has elapsed, information on the intensity of the signal attributable the test substance, information on the amount of the test substance and the like in the memory 123b. The processor 123a also has the function of generating image information for display on the display unit 125 from the information on the measured value of the intensity of the unprocessed signal including the signal attributable to the test substance, the information on the measured value of the background signal in the absence of the test substance, information on the calculated value of the intensity of the background signal at an optional time after a predetermined time has elapsed, information on the intensity of the signal attributable to the test substance, information on the amount of the test substance and the like. The processor 123a has a function of outputting the generated image information to the display unit 125.
The memory 123b temporarily stores information on the measured value of the intensity of the unprocessed signal including the signal attributable to the test substance, information on the measured value of the background signal in the absence of the test substance, information on the calculated value of the intensity of the background signal at an optional time point after a predetermined time has elapsed, information on the intensity of the signal attributable to the test substance, information on the amount of the test substance and the like. The memory 123b also may store information on a power approximate expression, an exponential approximate expression, a linear approximation expression or a combination thereof, information on the in-vivo concentration of a substance correlated with the in-vivo concentration of the test substance, information on a correction equation and the like.
The display unit 125 displays an image based on the image information output from the processor 123a. For example, information on the amount of the test substance together with information at an optional point in time after a predetermined time has elapsed are displayed on the display unit 125.

Test Substance Continuous Monitoring Method Procedure

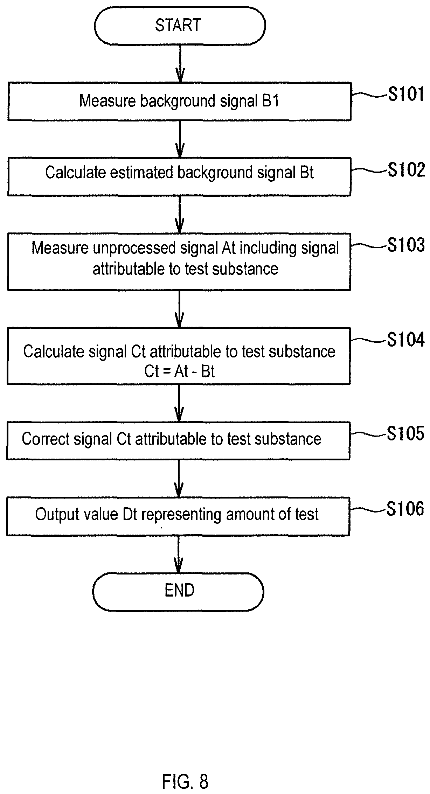

Next, the processing procedure of the test substance continuous monitoring method using the continuous monitoring system 10 will be described with reference to the drawings. FIG. 8 is a flowchart showing the processing procedure of the test substance continuous monitoring method according to the first embodiment.

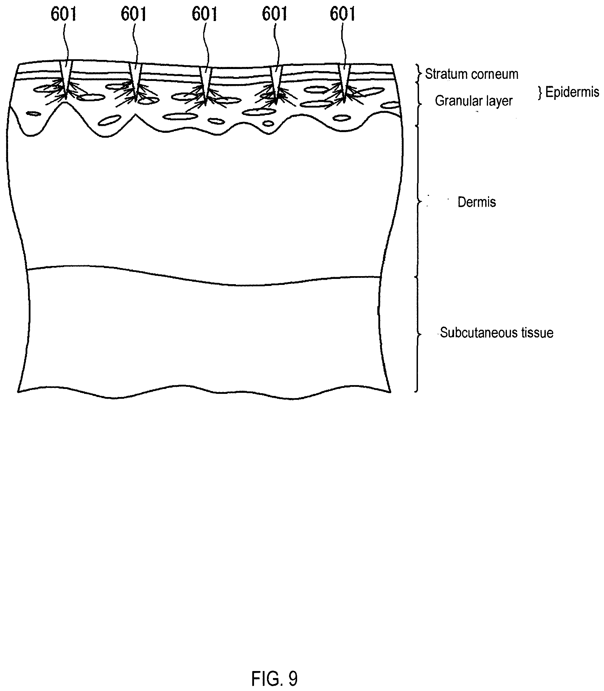

Before step S101, the user first applies interstitial fluid derivation promotion processing to the skin of the forearm. Examples of methods for performing the interstitial fluid derivation promotion treatment include, but are not limited to, a method of forming an interstitial fluid extraction region containing micropores in the skin using a puncture tool equipped with a fine needles; a method of treating the skin to form an interstitial fluid extraction region by microinvasive treatment of the skin by a tape, friction, polishing, ultrasonic irradiation. From the perspective of suppressing damage to the skin, it is preferable to use a method of forming an interstitial fluid extraction region using a puncture tool equipped with a microneedle and a method of forming a interstitial fluid extraction region by polishing or ultrasonic waves. In the case of performing ultrasonic irradiation, the frequency of the ultrasonic wave is preferably 10 kHz to 500 kHz, more preferably 20 kHz to 200 kHz, and most preferably 30 to 150 kHz. The case in which interstitial fluid derivation promotion processing is performed by a method of forming an interstitial fluid extraction region using a puncture tool equipped with a microneedle will be described below. As shown in FIG. 9, the user uses a puncture needle equipped with a microneedle to form a interstitial fluid extraction region including micropores 601 in the epidermis of the forearm. Next, the user closes the fine holes 601 in the interstitial fluid extraction region with a tape or the like. The user also adheres the collection member 40 onto this tape. Next, the sensor assembly 20 is installed on the collection member 40.

In step S101, the user first activates the sensor assembly 20. In the sensor assembly 20, the processor 115a of the control unit 115 then causes the electrochemical sensor 30 to measure the background signal B1 for a predetermined time. In step S101, the change over time of the intensity of the background signal for a predetermined time is measured. Examples of methods of measuring the background signal B1 include, but are not particularly limited to, an electrochemical measurement method such as a chronoamperometry method, a cyclic voltammetry method, a chronocoulometry method and the like.

Measurement of the background signal B1 preferably is performed during the warm-up time of the electrochemical sensor 30 of the sensor assembly 20. Normally, in the case of performing measurement using an electrochemical sensor, a certain amount of time is required until the current signal flowing in the electrochemical sensor is stabilized and the measured value is stabilized after energization. Therefore, the electrochemical sensor is energized without actual measurement during the period from when the electrochemical sensor is energized until the current signal flowing into the electrochemical sensor is stabilized. However, the inventors of the present invention discovered can effectively utilize the warming-up time of the electrochemical sensor 30 can be effectively utilized and the amount of the test substance can be accurately calculated by measuring the background signal B1 at the warming-up time of the electrochemical sensor 30. Note that the "warming-up time" is the time from the application of the voltage to the electrochemical sensor until the measured value becomes steady. The measurement time of the change over time of the intensity of the background signal B1 is preferably 20 minutes or more, and more preferably 30 minutes or more from the perspective of obtaining a calculated value of the intensity of the background signal with high accuracy. The measurement time of the change over time of the intensity of the background signal B 1 preferably is 90 minutes or less, and more preferably 60 minutes or less from the perspective of preventing a reduction in the quality of life of the subject. In step S101, the processor 115a acquires information on the intensity of the background signal B1 measured by the electrochemical sensor 30. Subsequently, the processor 115a stores the information on the acquired intensity of the background signal B1 in the memory 115b.

The background signal B1 can be measured by applying a voltage to the electrochemical sensor 30 in a state in which the collection member 40 that does not include the test substance is attached to the electrochemical sensor 30.

Next, in step S102, the control unit 115 of the sensor assembly 20 calculates the estimated background signal Bt which is the background signal at the time of measurement of the test substance. In step S102, the processor 115a of the control unit 115 first obtains from the memory 115b information on the intensity of the background signal B1 and information on the power approximate expression, the exponential approximation formula, the linear approximation formula, or a combination thereof. Next, the processor 115a performs the best adaptation to the change over time of the intensity of the background signal B1 for a predetermined time from the power approximate expression, the exponential approximation expression, the linear approximation expression or combinations thereof which are estimation formulae.

The following expression is an example of the estimation equation, but is not particularly limited. (1) $y = Ax^B$ (where A and B are constants, y is a calculated value of the background signal and x is the measurement time), (2) $y = Ax^B$, (3) $y = Ax^B + Cx + D$ (where A, B and C are constants, y is the calculated value of the background signal and x is the measurement time), (4) $y = Ae^{Bx}$ (where A and B are constant, y is the calculated value of the background signal and x is the measurement time), (5) $y = Ae^{Bx} + C$ (where A, B and C are constants, y is the calculated value of the bu cloud signal, (6) $y = Ae^{Bx} + Cx + D$ where A, B, C and D are constants, y is a background signal, X is the measurement time).

The processor 115a causes the estimation equation to fit with respect to the temporal change of the intensity of the background signal and obtains a constant in the estimation equation. Thereafter, the processor 115a calculates the estimated background signal Bt in the measurement time of the test substance according to the estimation formula. The processor 115a stores the information on the calculated estimated background signal Bt in the memory 115b.

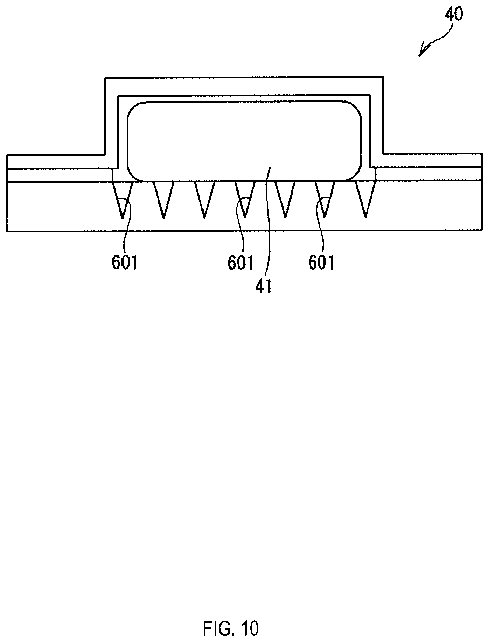

In step S103, the processor 115a of the control unit 115 then causes the electrochemical sensor 30 to measure the unprocessed signal At including the signal attributable to the test substance. In step S103, the user first removes the tape between the collection member 40 and the interstitial fluid extraction area including the microhole 601 of the forearm. Specifically, the user removes the sensor assembly 20, the collection member 40 and the tape from the forearm. As shown in FIG. 10, the user then adheres the collection member 40 to the tissue extracting area so that the extraction medium and the interstitial fluid extraction area including the microhole 601 of the forearm are in contact with each other. Next, the user mounts the sensor assembly 20 on the collection member 40. Subsequently, in step S103, the processor 115a causes the electrochemical sensor 30 to measure the unprocessed signal At including the signal attributable to the test substance. Examples of a method for measuring the untreated signal At including a signal attributable to the test substance include, but are not limited to, an electrochemical measurement method such as a chronoamperometry method, a cyclic voltammetry method, a chrono-coulometry method and the like. In step S103, the processor 115a acquires information on the intensity of the unprocessed signal At including the signal attributable to the test substance measured by the electrochemical sensor 30. Thereafter, the processor 115a stores the information of the unprocessed signal At including the signal derived from the acquired test substance in the memory 115b.

Next, in step S104, the processor 115a of the control unit 115 calculates the signal Ct attributable to the test substance. In step S104, the processor 115a of the control unit 115 first acquires from the memory 115b the information of the unprocessed signal At including the signal attributable to the test substance, and the information of the intensity of the estimated background signal Bt. Next, the processor 115a subtracts the value of the intensity of the estimated background signal Bt from the measured value of the intensity of the unprocessed signal At including the signal attributable to the test substance. As a result, the processor 115a calculates the signal Ct attributable to the test substance. The processor 115a stores the information of the signal Ct attributable to the calculated test substance in the memory 115b.

It should be noted that the calculation of the signal Ct caused by the test substance in step S104 also may be executed by the terminal 60 instead of being executed by the processor 115a of the sensor assembly. In this case, before step S104, the processor 115a outputs the information of the unprocessed signal At including the signal attributable to the test substance, and the information of the intensity of the estimated background signal Bt to the terminal 60.

Next, in step S105, the processor 115a executes the correction of the signal Ct attributable to the test substance. In step S105, the processor 115a first acquires the information on the signal Ct attributable to the test substance, and information on the in-vivo concentration of the substance correlated with the in-vivo concentration of the test substance from the memory 115b. Next, the processor 115a uses the in-vivo concentration of the substance correlated with the in-vivo concentration of the test substance, the correction formula and the like, so that the signal Ct attributable to the test substance is converted to a value Dt indicating the amount of the test substance.

The in-vivo concentration of the substance correlated with the in-vivo concentration of the test substance may be, for example, the blood concentration of the test substance, the concentration of other substances correlated with the test substance in the interstitial fluid, the blood concentration of other substances correlated with the test substance in the blood and the like, but the correlated substance is not particularly limited. From the perspective of improving accuracy, the in-vivo concentration of the substance correlated with the in-vivo concentration of the test substance preferably is the blood concentration of the test substance. The blood concentration of the test substance can be measured by, for example, a self-administered blood glucose measuring device or the like. When the blood concentration of the test substance is used as the in-vivo concentration of the substance correlated with the in-vivo concentration of the test substance, the blood concentration of the test substance is preferably measured within the measurement time of the background signal B1 from the viewpoint of more accurately estimating the amount of the test substance.

The correction formula is not particularly limited insofar as the concentration of the test substance can be obtained. In step S105, for example, the blood concentration of the test substance used for correction is a "value obtained by subtracting the estimated background current value (corresponds to the intensity of the estimated background signal Bt) from the measured value of the unprocessed current signal a1 including the current value attributable to the test substance (corresponds to the intensity of the unprocessed signal At including the signal attributable to the test substance), that is it can be regarded as equal to the current value attributable to the test substance. In this case, a determination is first made regarding the ratio (= the blood concentration of the test substance / the measured value of the current signal) for converting the measurement value of the unprocessed current signal a1 including the current signal attributable to the test substance into the blood concentration of the test substance. Next, the processor 115a calculates the amount of the test substance from the current value attributable to the test substance. In step S105, for example, the amount of the test substance may be calculated using the variable amount of the current value taking into consideration the shrinkage of the micropores in the skin and the deterioration over time of the electrochemical sensor and the like as a correction formula, that is:

[amount of test substance (calculated amount)] = ([in-vivo concentration of a substance correlating with the in-vivo concentration of the test substance] × [current value attributable to the test substance]) / [variation amount of current value]. In step S105,

In step S105, the amount of variation of the current value also can be calculated by the aforementioned power approximation, exponential approximation, linear similarity or a combination thereof. In step S105, the processor 115a stores the information of the value Dt indicating the amount of the test substance in the memory 115b.

Thereafter, in step S106, the processor 115a outputs the information of the value Dt indicating the amount of the test substance stored in the memory in step S105 to the terminal 60.

In the test substance continuous monitoring method of the first aspect, after applying the interstitial fluid derivation promotion process to the forearm skin, the measurement of the background signal B1 by the sensor assembly 20, the calculation of the estimated background signal Bt, and the measurement of the unprocessed signal At including the signal attributable to the test substance are performed. On the other hand, the interstitial fluid derivation promotion process also may be performed after measurement of the background signal B1 by the sensor assembly 20 and calculation of the estimated background signal Bt. In this case, the measurement of the background signal B1 can be performed by bringing the sensor assembly 20 and the collection member 40 into contact without placing the sensor assembly 20 and the collection member 40 on the forearm.

In the test substance continuous monitoring method according to the first aspect, a background signal at the time of measurement of an unprocessed signal including a signal attributable to a test substance is determined based on a background signal at a point within a predetermined time. For example, the background signal at the time the background signal is stabilized may be subtracted from the unprocessed signal including the signal attributable to the test substance. Also, the timing of the measurement of the background signal need not be before the measurement of the unprocessed signal including the signal attributable to the test substance, and may be after the measurement of the unprocessed signal including the signal attributable to the test substance.

In the test substance continuous monitoring method of the first aspect, the measurement of the background signal B1 and the calculation of the estimated background signal Bt are carried out when carrying out the test substance continuous monitoring method. On the other hand, the measurement of the background signal B1 and the calculation of the estimated background signal Bt may be performed in advance in the manufacturing process of the continuous monitoring system 10 (continuous monitoring method of the second aspect). In this case, information of the estimation expression and information of the estimated background signal Bt can be stored in the memory 115a of the control unit 115 of the sensor assembly 20 in advance. In this case, the user also can immediately perform the measurement of the test substance using the continuous monitoring system 10. Hereinafter, the continuous monitoring method of the second aspect will be described.

In step S201 of the continuous monitoring method according to the second aspect, the processor 115a of the control unit 115 of the sensor assembly 20 causes the electrochemical sensor 30 to measure the unprocessed signal At including the signal attributable to the test substance. Step S201 is the same as step S 103 in the continuous monitoring method of the first aspect.

Next, in step S202, the processor 115a executes a call of the estimated background signal Bt stored in the memory 115a and acquires these pieces of information.

Thereafter, in steps S203 to S205, the processor 115a executes the calculation of the signal Ct attributable to the test substance, the correction of the signal Ct attributable to the test substance, and the output of the value Dt representing the test substance. Steps S203 to S205 are the same as steps S 104 to S 106 in the continuous monitoring method of the first aspect.

Examples

Description of Terms

PEN: polyethylene naphthalate
PBS: phosphate buffered saline
PVA: polyvinyl alcohol

Example 1

In the following, according to Example 1 and Comparative Example 1, the influence of the presence or absence of calculation of the estimated background current value at an optional time point after a predetermined time has elapsed from the start of the measurement of the background current signal, and the presence or absence of using the estimated background current value were examined.

(1) Electrode Layer Formation

A working electrode (diameter: 9 mm) configured by a platinum thin film and a counter electrode configured by a platinum thin film were formed on one surface of a PEN film by vapor deposition. In addition, a silver/silver chloride ink was applied to the surface of the PEN film on the working electrode and the counter electrode side and dried to form a reference electrode made of silver/silver chloride. An electrode substrate was obtained in this way.

(2) Enzyme Layer Formation

12.7 $\mu$L of an enzyme solution [PBS solution containing 0.25% by mass glutaraldehyde, 42.984 mg/dL bovine serum albumin, 1.5225 U/mL glucose oxidase, and 0.5 U/mL mutarotase] was dripped on the working electrode of the electrode substrate obtained in Example 1. Next, the electrode substrate was allowed to stand in an environment kept at 25°C at a relative humidity of 30% to dry the enzyme solution. In this way an enzyme layer was formed on the working electrode to obtain a glucose sensor.

(3) Hydrogel Preparation

A hydrogel (lateral: 20 mm, longitudinal: 20 mm, thickness: 0.2 mm) of PVA was obtained by irradiating PVA aqueous solution A (composition: 12% (w/w) PVA, 2% (w/w) potassium chloride, and 86% (w/w) water) with an electron beam having a dose of 25 kGr.

(4) Formation of Interstitial Fluid Extraction Area

The human forearm of healthy subjects was disinfected with ethanol. Using a puncture tool equipped with a fine needle (manufactured by SYSMEX CORPORATION), an interstitial fluid extraction region with a diameter of 8 mm containing fine pores was formed on the forearm.

(5) Suppression of Exudation of Interstitial Fluid

A mending tape (manufactured by 3M Co., Ltd.) was adhered on the interstitial fluid extraction region formed in Example 1 (4) to suppress exudation of interstitial fluid from micropores in the interstitial fluid extraction region.

(6) Application of Hydrogel and Attachment of Glucose Sensor

The hydrogel obtained in Example 1 (3) was further attached to a mending tape (manufactured by 3M Co., Ltd.) attached to the interstitial fluid extraction region in Example 1 (5). Subsequently, a glucose sensor was attached to the hydrogel on the interstitial fluid extraction region so that the working electrode, the reference electrode and the counter electrode of the glucose sensor respectively came into contact with the hydrogel on the interstitial fluid extraction region. In this way the glucose sensor was attached to the forearm. A mending tape (manufactured by 3M Co., Ltd.), a hydrogel and a glucose sensor are superimposed in this order on the interstitial fluid extraction region where micropores were formed in the human forearm.

(7) Background Current Signal Measurement

In Example 1 (6), the glucose sensor attached to the forearm was connected to a potentiostat (trade name: ALS832b, manufactured by BAS, Inc.). Using a reference electrode as a reference, a voltage of 0.45 V was applied to the working electrode of the glucose sensor. A background current signal flowing between the working electrode and the counter electrode was measured for 2690 seconds.

(8)Measurement of Unprocessed Current Signal Including Current Signal Attributable to Glucose in Hydrogel

After measuring the background current signal in Example 1 (7), the glucose sensor, hydrogel, and mending tape (manufactured by 3M Co., Ltd.) were removed from the interstitial fluid extraction region of the forearm. Immediately thereafter, a hydrogel was applied on the interstitial fluid extraction area so as to cover the interstitial fluid extraction area of the forearm. Next, the glucose sensor was adhered to the hydrogel on the interstitial fluid extraction area so that the center position of the interstitial fluid extraction area aligned with the center position of the working electrode of the glucose sensor while the working electrode, the reference electrode, and the counter electrode of the glucose sensor were in contact with the hydrogel on the interstitial fluid extraction area. In this way the glucose sensor was attached to the forearm. Simultaneously with the mounting of the glucose sensor, the glucose sensor started the measurement of the unprocessed current signal a1 including a current signal attributable to the glucose test substance in the hydrogel. Measurement of the current signal by the glucose sensor was performed at two-second intervals. In this way a measured value (hereinafter also referred to as "current measured value A1") of the unprocessed current signal a1 including a current signal attributable to glucose was obtained. Note that in the following description the current measurement value A1 at the measurement time point of the elapsed time t from the start of the measurement of the unprocessed current signal including the current signal attributable to glucose is also referred to as "current measurement value A1t".

(9) Blood Glucose Level Measurement

A blood glucose self-administered measurement device (trade name: Freestyle, manufactured by Nipro Co., Ltd.) was used at the time of 3600 seconds and 23700 seconds from the start of the measurement of the unprocessed current signal including the current signal attributable to glucose in Example 1 (8) to measure the blood glucose level of the blood obtained by puncturing the fingertip (hereinafter also referred to as "calibration blood glucose level $G_{3600}$" and "calibration blood glucose level $G_{23700}$"). Using the current value at 3600 seconds after the start of measurement and the current value at 23700 seconds elapsed, the formula (Ia) was obtained.

$$y = -\ 0.002 \text{ x } t + 87.689 \text{ (Ia)}$$

In the formula (Ia), t represents the elapsed time from the start of the measurement of the unprocessed current signal including the current signal attributable to glucose.
This formula was used to obtain the estimated blood glucose level from the current measurement value A1t obtained in Example 1 (8).
Blood glucose levels were measured at intervals of 900 to 1800 seconds using a blood glucose self-administered measurement device from 4500 seconds after the start of measurement of an unprocessed current signal including a current signal attributable to glucose in Example 1 (8). Note that the blood glucose level at the measurement time point t from the start of the measurement of the unprocessed current signal including the current signal attributable to glucose is also referred to as "measured blood glucose level Gt". The measured blood glucose level Gt was used for comparison with the estimated blood glucose level and was used as the reference blood glucose level.

(10) Calculation of Estimated Background Current Value

An approximation curve was obtained by performing power approximation on the background current value obtained in Example 1 (7). Based on the obtained approximation curve, the formula (IIa) was used as a calculation formula of the background current value at the measurement time point of elapsed time t from the start of measurement of the unprocessed current signal attributable to glucose in Example 1 (8) (hereinafter referred to as "estimated back Ground current value Bt "), that is, formula (IIa):

$$\text{(Estimated background current value Bt)} = 625.3 \times t - 0.399 \text{ (IIa)}$$

(wherein, in the formula (IIa), t represents the elapsed time from the start of the measurement of the unprocessed current signal including the current signal attributable to glucose) was obtained. Based on the formula (IIa), an estimated background current value Bt was obtained.

(11) Estimated Blood Glucose Level Calculation

The current value Ct attributable to the test substance was calculated using the current measurement value A1t obtained in Example 1 (8) and the estimated background current value Bt at the measurement time point corresponding to the current measurement value A1t, and based on the formula (III).

(Current value Ct attributable to the test substance) = (current measured value A1t - estimated background current value Bt) (III)

The measurement time point of the current signal in Example 1 (8) was divided at intervals of 5 minutes, and the average value of the current value Ct attributable to the test substance obtained within one delimited period (5 minutes) was calculated.

In order to eliminate the temporal deviation until the blood glucose level of the blood is reflected in the blood glucose value estimated from the amount of glucose in the interstitial fluid, a time correction of 152 seconds was performed on the average value of the current value Ct attributable to the test substance. Next, the estimated blood glucose level Dt was calculated based on the formula (IVa).

(Estimated blood glucose level Dt) = (mean blood glucose level $\times$ i) / (- 0.002 $\times$ t + 87.689)
(IVa)

Note that in the formula (IVa) the average blood glucose level is the average value of the calibration blood glucose level $G_{3600}$ and the calibration blood glucose level $G_{23700}$, where t is the elapsed time from the start of measurement of the background current signal, and i represents the current value Ct attributable to the test substance, and [- 0.002 $\times$ t + 87.689] corresponds to the straight line represented by the formula (Ia).

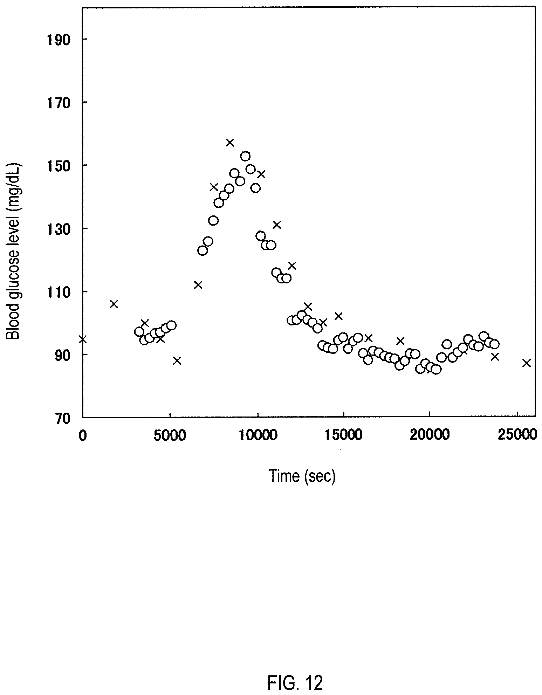

Thereafter, the changes over time in the estimated blood glucose level and the measured blood glucose level were examined. FIG. 12 shows the result of examining the change over time in blood glucose level in Example 1. In the drawing, open circles indicate change over time in the estimated blood glucose level, and crosses show change over time in the measured blood glucose level. Next, in FIG. 12, the accuracy of the estimated blood glucose level was evaluated by error grid analysis using the estimated blood glucose level and the measured blood glucose level at the same time point.

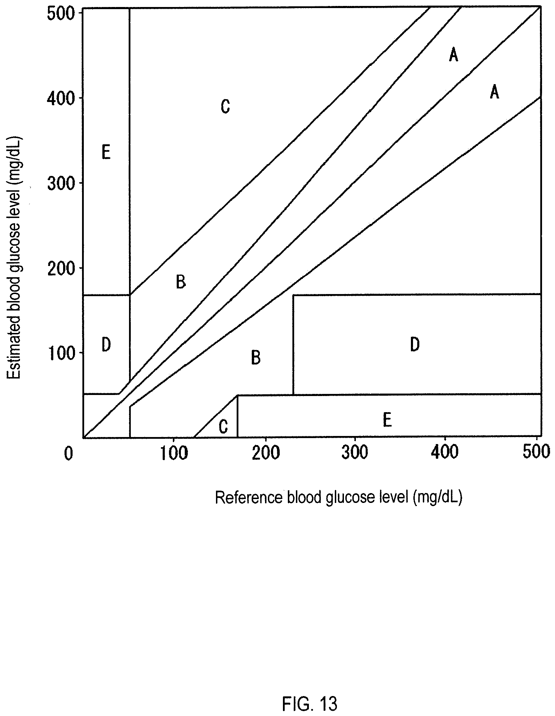

Note that in the error grid analysis the accuracy of the estimated blood glucose level is classified into the areas A to E shown in FIG. 13 and evaluated by comparing the estimated blood glucose level and the reference blood glucose level which is the measured blood glucose level. In FIG. 13, the significance of each region is as follows.

Significance of Each Area in Error Grid Analysis

Region A: Clinically accurate, leads to correct medical judgment. That is, region A has clinical accuracy.
Region B: leads to benign judgment or treatment unnecessary. That is, although it is inferior to A, the accuracy has no clinical problem.
Region C: Over-corrects normal blood glucose level. That is, there is a risk of performing unnecessary treatment.
Region D: Hyperglycemic value or a hypoglycemic value may be overlooked.
Region E: Leads to incorrect and dangerous treatment decisions.

Therefore, the accuracy of the estimated blood glucose level is usually judged to have a precision with no practical problem if the plot of the estimated blood glucose level falls within the areas A and B in the error grid analysis.

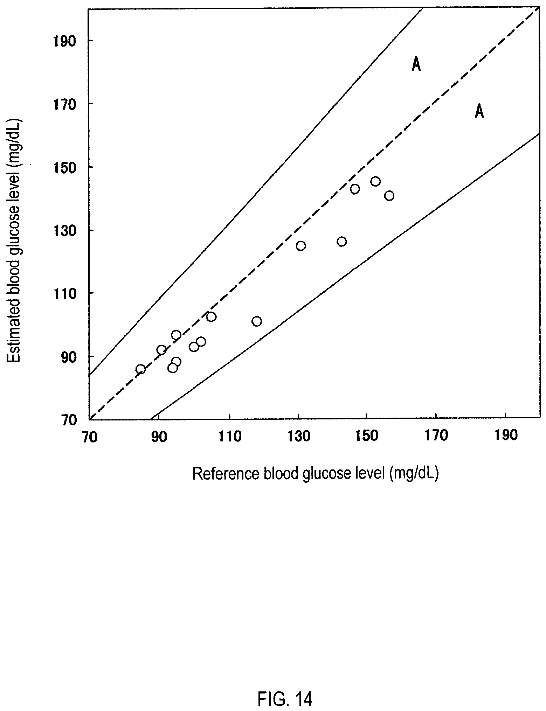

The result of evaluating the estimated blood glucose level by error grid analysis in Example 1 is shown in FIG. 14. In FIG. 14, A indicates the area A in the error grid analysis.

(12) Results

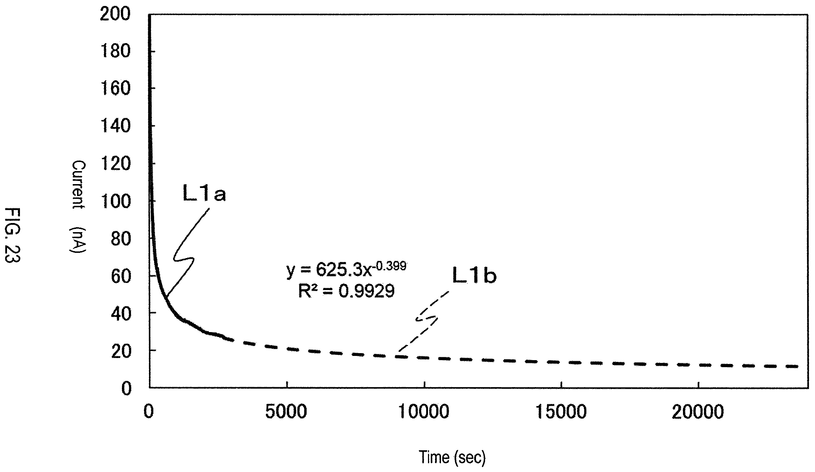

As shown in FIG. 23, the background current signal measured in Example 1 (7) shows the change over time of the curve L1a represented by y = 625.3 $\times$ $t^{-0.399}$. The estimated background current value calculated in Example 1 (10) also shows the change over time of the curve L1b represented by y = 625.3 $\times$ $t^{-0.399}$ even after the measurement time of the background current signal obtained in Example 1 (7). Accordingly, it can be understood that the intensity of the background signal at the time of measurement of the unprocessed current signal including the current signal attributable to glucose can be calculated by fitting the equation of power approximation, exponential approximation, linear approximation, or a combination thereof to the change over time of the measured background current value.

From the results shown in FIG. 14, it was found that all the 14 plots of the estimated blood glucose level obtained in Example 1 were within the area A. When calculating the variation of the estimated blood glucose level relative to the reference blood glucose level (mean absolute relative difference (hereinafter referred to as "MARD"), the MARD was 6.2%. These results suggested that an highly accurate estimated blood glucose level can be obtained by using the estimated background current value calculated using the background current value measured by the glucose sensor used for glucose measurement.

Comparative Example 1

In Comparative Example 1, the estimated blood glucose level Dt was calculated without measuring the background current value, calculating the estimated background current value, nor calculating the current value Ct attributable to the test substance.

The measurement time point of the current signal in Example 1 (8) was divided at intervals of 5 minutes, and the average value was calculated for the current measurement value A1t obtained in Example 1 (8) within one delimited period (5 minutes). In addition, the same operation as in Example 1 (9) was performed to obtain the calibration blood glucose level $G_{3600}$, the calibration blood glucose level $G_{23700}$, and the measured blood glucose level Gt. The formula (Ib) was obtained using the current value at 3600 seconds after the start of measurement and the current value at 23700 seconds elapsed.

$$y = -0.0026 \times t + 113.42 \text{ (Ib)}$$

Note that in the formula (Ib) t represents the elapsed time from the start of the measurement of the unprocessed current signal including the current signal attributable to glucose.

A time correction of 152 seconds was performed on the average value of the current measurement value A1t. Next, the estimated blood glucose level Dt was calculated based on the formula (IVb).

$$\text{(Estimated blood glucose level Dt)} = (\text{mean blood glucose level} \times i) / (-0.0026 \times t + 113.42)$$
$$\text{(IVb)}$$

Note that in the formula (IVb) the average blood glucose level is the average value of the calibration blood glucose level $G_{3600}$ and the calibration blood glucose level $G_{23700}$, where t is the elapsed time from the start of measurement of the unprocessed current signal including the current signal attributable to glucose, and i represents the current value Ct attributable to the test substance, and [- 0.0026 × t + 113.42] corresponds to the straight line represented by the formula (Ib).

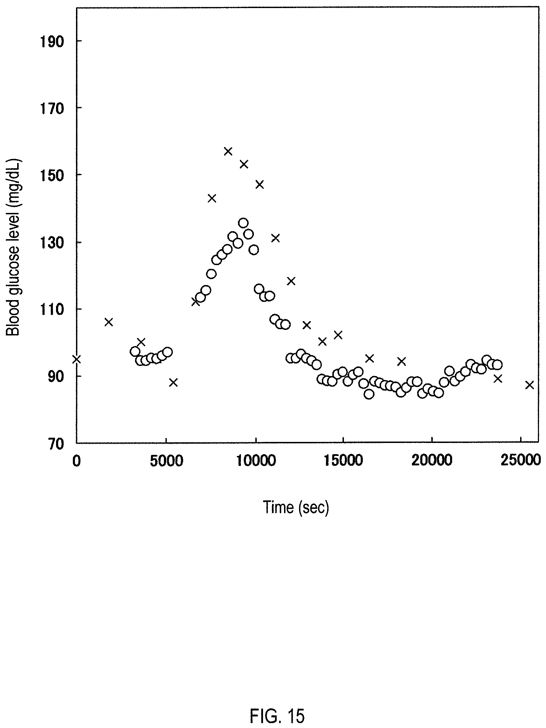

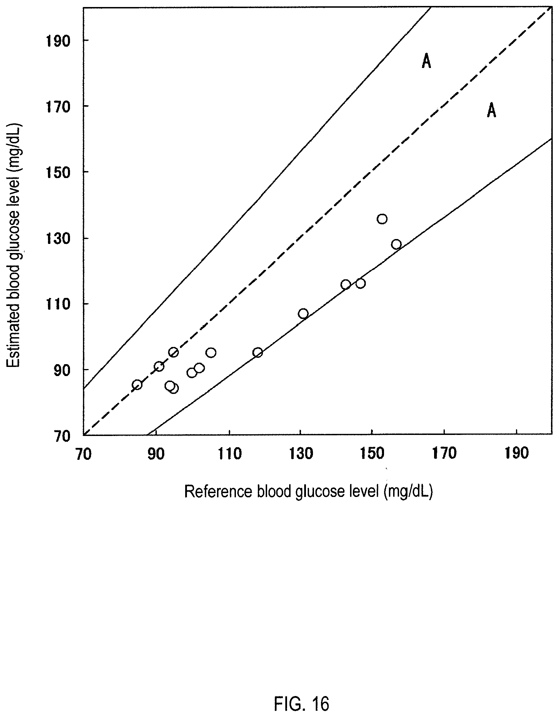

Thereafter, the changes over time in the estimated blood glucose level and the measured blood glucose level were examined. FIG. 15 shows the result of examining the change over time in blood glucose level in Comparative Example 1. In the drawing, open circles indicate change over time in the estimated blood glucose level, and crosses show change over time in the measured blood glucose level. Next, in FIG. 15, the accuracy of the estimated blood glucose level was evaluated by error grid analysis using the estimated blood glucose level and the measured blood glucose level at the same time point. The result of evaluating the estimated blood glucose level by error grid analysis in Comparative Example 1 is shown in FIG. 16. In FIG. 16, A indicates the area A in the error grid analysis.

From the results shown in FIG. 16, it was found that all the 13 plots of the estimated blood glucose level obtained in Comparative Example 1 were within the area A. The MARD also was 17.7%. In Example 1 and Comparative Example 1, the point that the estimated background current value was not calculated at an optional time after a predetermined time had elapsed from the start of the measurement of the background current signal, and the point that the estimated background current value was not used are different. These results suggested that an estimated blood glucose level with high accuracy can be obtained according to the method of Example 1, since the estimated background current value was calculated at an optional time after a predetermined time had elapsed from the start of the measurement of the background current signal, and the estimated background current value was used.

Example 2

Below, the influence on the accuracy of the estimated blood glucose level was examined according to whether or not the estimated background current value was used and whether or not the estimated background current level was calculated at an optional time point after a predetermined time had elapsed after the start of measurement of the background current signal when the timing of the measurement of the calibration blood glucose level and the measured blood glucose level was changed according to Example 2 and comparative Example 2.

(1) Electrode Layer Formation

The same operation as in Example 1 (1) was carried out to obtain an electrode substrate.

(2) Enzyme Layer Formation

The same operation as in Example 1 (2) was carried out to obtain a glucose sensor.

(3) Hydrogel Preparation

The same operation as in Example 1 (3) was carried out to obtain a PVA hydrogel.

(4) Formation of Interstitial Fluid Extraction Area

The same operation as in Example 1 (4) was carried out to form the interstitial fluid extraction area.

(5) Suppression of Exudation of Interstitial Fluid

The same operation as in Example 1 (5) was carried out to suppress exudation of interstitial fluid from micropores in the interstitial fluid extraction area.

(6) Application of Hydrogel and Attachment of Glucose Sensor

The same operation as in Example 1 (6) was carried out, and application of a hydrogel and attachment of a glucose sensor were accomplished.

(7) Background Current Signal Measurement

A background current signal was measured in the same manner as in Example 1 (7) except that a small potentiostat (manufactured by Fuso Manufacturing Co., Ltd.) was used.

(8)Measurement of Unprocessed Current Signal Including Current Signal Attributable to Glucose in Hydrogel

The same operation as in Example 1 (8) was carried out to obtain current measurement value A1t.

(9) Blood Glucose Level Measurement

The same operation as in Example 1 (9) was performed to obtain a calibration blood glucose level $G_{2700}$ and a calibration blood glucose level $G_{29100}$ except that measurement of the calibration blood glucose level was made at the time of 2700 seconds elapsed and at the time of 29100 seconds elapsed from the start of measurement of the unprocessed current signal including the current signal attributable to glucose in Example 2 (8).
The same operation as in Example 1 (9) was carried out to obtain a measured blood glucose level Gt except that the blood glucose level was measured using a self-administered glucose measurement device at 900 to 1800 second intervals after 3600 seconds had elapsed after the start of the measurement of the unprocessed current signal including the current signal attributable to glucose in Example 2 (8).

(10) Calculation of Estimated Background Current Value

An approximation curve was obtained by performing power approximation on the background current value obtained in Example 2 (7). Based on the obtained approximate curve, formula (IIb) was obtained as an equation for calculating the estimated background current value Bt.

$$\text{(Estimated background current value Bt)} = 3204.9 \times t^{-0.507} \text{ (IIb)}$$

Note that in the formula (Ia) t represents the elapsed time from the start of the measurement of the unprocessed current signal including the current signal attributable to glucose.

Based on the formula (IIb), an estimated background current value Bt was obtained.

(11) Estimated blood glucose Level Calculation

The current value Ct attributable to the test substance was calculated using the current measurement value A1t obtained in Example 1 (8), and the estimated background current value Bt at the measurement time point corresponding to the current measurement value A1t, and based on the formula (III). The measurement time point of the current signal in Example 1 (8) was divided at intervals of 5 minutes, and the average value of the current value Ct attributable to the test substance obtained within one delimited period (5 minutes) was calculated.

A time correction of 454 seconds was performed on the average value of the current value Ct attributable to the test substance. Next, the estimated blood glucose level Dt was calculated based on the formula (IVc).

$$\text{(Estimated blood glucose level Dt)} = \text{(mean blood glucose level} \times i) / (150.151 \times \exp(-0.0027 \times t) - 0.00143 \times t + 138.806)$$
$$\text{(IVc)}$$

Note that in the formula (IVc) the average blood glucose level is the average value of the calibration blood glucose level $G_{2700}$ and the calibration blood glucose level $G_{29100}$, where t is the elapsed time since the measurement start of the unprocessed current signal including the current signal attributable to glucose, i represents the current value Ct attributable to the test substance, and $[150.151 \times \exp(-0.0027 \times t) - 0.00143 \times t + 138.806]$ corresponds to the attenuation curve calculated from the variation of the current measurement value A1 since, even if the blood glucose level is constant, it is assumed that the current measurement value A1 attenuates with the passage of time due to the influence such as shrinkage of the micropore and degradation over time of the electrochemical sensor.

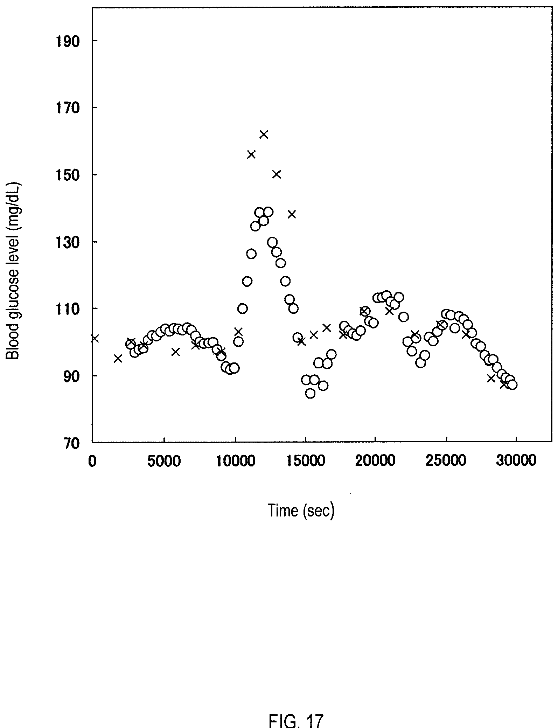

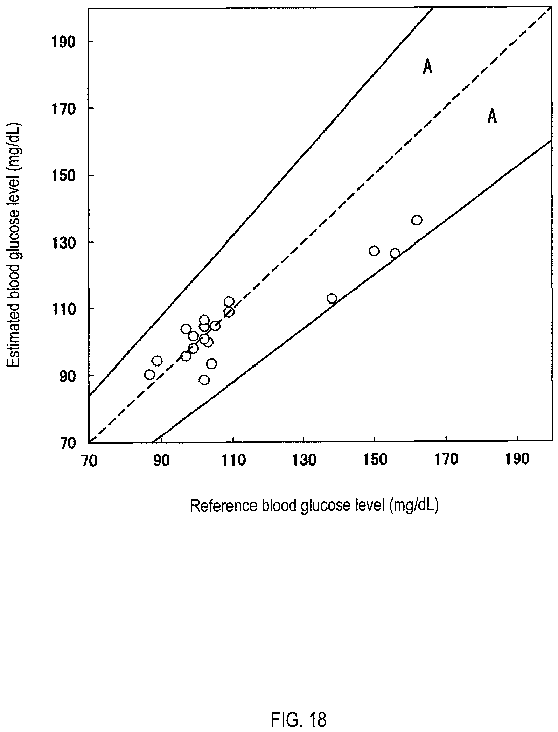

Thereafter, the changes over time in the estimated blood glucose level and the measured blood glucose level were examined. FIG. 17 shows the result of examining the change over time in blood glucose level in Example 2. In the drawing, open circles indicate change over time in the estimated blood glucose level, and crosses show change over time in the measured blood glucose level. Next, in FIG. 17, the accuracy of the estimated blood glucose level was evaluated by error grid analysis using the estimated blood glucose level and the measured blood glucose level at the same time point. The result of evaluating the estimated blood glucose level by error grid analysis in Example 2 is shown in FIG. 18. In FIG. 18, A indicates the area A in the error grid analysis.

(12) Results

From the results shown in FIG. 18, it was found that all the 19 plots of the estimated blood glucose level obtained in Example 2 were within the area A. The MARD also was 6.7%. These results suggested that a highly accurate estimated blood glucose level can be obtained using the estimated background current value calculated using the background current value measured with the glucose sensor used for the measurement of glucose even when the measurement timing of the calibration blood glucose level and the measured blood glucose level Gt is changed.

Comparative Example 2

The same operation as in Comparative Example 1 was carried out to calculate the estimated blood glucose level Dt except that the measured current value A1t obtained in Example 2 (8) was used, the measurement time of the calibration blood glucose level was at the time of 2700 seconds elapsed and 29100 seconds elapsed from the start of measurement of the unprocessed current signal including the current signal attributable to glucose, and the measurement of the measured blood glucose level Gt was performed at intervals of 900 to 1800 seconds after 3600 seconds had elapsed from the start of the measurement of the unprocessed current signal including the current signal attributable to glucose. Note that in Comparative Example 2 the formula for calculating the estimated blood glucose level Dt was the formula represented by the formula (IVd).

$$\text{(Estimated blood glucose level Dt)} = \text{(mean blood glucose level} \times i) / (186.5161 \times \exp(-0.002 \times t) - 0.00093 \times t + 150).$$
$$\text{(IVd)}$$

Note that in the formula (IVd) the average blood glucose level is the average value of the calibration blood glucose level $G_{2700}$ and the calibration blood glucose level $G_{29100}$, where t is the elapsed time since the measurement start of the unprocessed current signal including the current signal attributable to glucose, i represents the current measurement value A1 at a measurement time point of elapsed time t after the measurement start of the unprocessed current signal including the current signal attributable to glucose, and [186.5161 $\times$ exp (- 0.002 $\times$ t) - 0.00093 $\times$ t + 150] corresponds to the attenuation curve calculated from the variation of the current measurement value since, even if the blood glucose level is constant, it is assumed that the current measurement value A1 attenuates with the passage of time due to the influence such as shrinkage of the micropore and degradation over time of the electrochemical sensor.

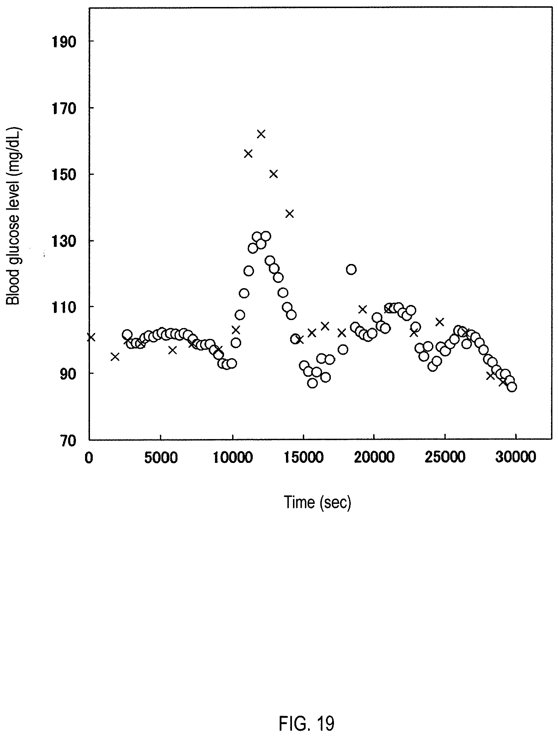

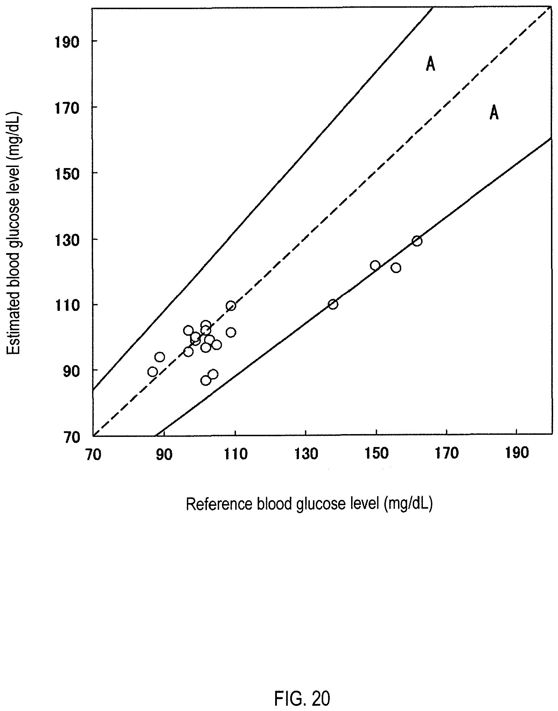

Thereafter, the changes over time in the estimated blood glucose level and the measured blood glucose level were examined. FIG. 19 shows the result of examining the change over time in blood glucose level in Comparative Example 2. In the drawing, open circles indicate change over time in the estimated blood glucose level, and crosses show change over time in the measured blood glucose level. Next, in FIG. 19, the accuracy of the estimated blood glucose level was evaluated by error grid analysis using the estimated blood glucose level and the measured blood glucose level at the same time point. The result of evaluating the estimated blood glucose level by error grid analysis in Comparative Example 2 is shown in FIG. 20. In FIG. 20, A indicates the area A in the error grid analysis.

From the results shown in FIG. 20, it was found that 17 of points the 19 plots of the estimated blood glucose level obtained in Comparative Example 2 were within the area A. The MARD also was 8.1 %. In Example 2 and Comparative Example 2, the point that the estimated background current value was not calculated at an optional time after a predetermined time had elapsed from the start of the measurement of the background current signal, and the point that the estimated background current value was not used are different. These results suggested that a highly accurate estimated blood glucose level can be obtained according to the method of Example 2, since the estimated background current value was calculated at an optional time after a predetermined time had elapsed from the start of the measurement of the background current signal, and the estimated background current value was used.

Example 3

In the following, the influence timing of the measurement of the background current signal on the accuracy of the estimated blood glucose level was examined when the timing of measuring the background current signal is changed from immediately before the measurement of the current signal due to glucose to one month before the measurement of the current signal due to glucose.

(1) Electrode Layer Formation

The same operation as in Example 1 (1) was carried out to obtain an electrode substrate.

(2) Enzyme Layer Formation

The same operation as in Example 1 (2) was carried out to obtain a glucose sensor.

(3) Hydrogel Preparation

The same operation as in Example 1 (3) was carried out to obtain a PVA hydrogel.

(4) Background Current Signal Measurement

The working electrode, the reference electrode and the counter electrode of the glucose sensor obtained in Example 3 (2) were brought into contact with the hydrogel obtained in Example 3 (3), respectively. Next, the glucose sensor was connected to a potentiostat (trade name: ALS 832 b, manufactured by BAS, Inc.). Using a reference electrode as a reference, a voltage of 0.45 V was applied to the working electrode of the glucose sensor. A background current signal flowing between the working electrode and the counter electrode was measured for 1428 seconds. Note that, during the measurement of the background current signal, drying of the hydrogel was suppressed by dripping PBS onto the hydrogel.

(5) Calculation of Estimated Background Current Value

An approximation curve was obtained by performing power approximation on the background current value obtained in Example 3 (4). Based on the obtained approximate curve, formula (IIc) was obtained as an equation for calculating the estimated background current value Bt.

$$(\text{Estimated background current value Bt}) = 1295.5 \times t^{-0.528} \text{ (IIc)}$$

Note that in the formula (IIc) t represents the elapsed time from the start of the measurement of the unprocessed current signal including the current signal attributable to glucose.

Based on the formula (IIc), an estimated background current value Bt was obtained.

(6) Formation of Interstitial Fluid Extraction Area

The same operation as in Example 1 (4) was carried out to form the interstitial fluid extraction area.

(7) Suppression of Exudation of Interstitial Fluid

One month after measurement of the background current signal, the same operation as in Example 1 (5) was performed to suppress exudation of interstitial fluid from micropores in the interstitial fluid extraction region.

(8) Measurement of Unprocessed Current Signal Including Current Signal Attributable to Glucose in Hydrogel

The hydrogel obtained in Example 3 (3) was adhered on the interstitial fluid extraction region so as to cover the interstitial fluid extraction region of the human forearm. Next, the glucose sensor was adhered to the hydrogel on the interstitial fluid extraction area so that the center position of the interstitial fluid extraction area aligned with the center position of the working electrode of the glucose sensor while the working electrode, the reference electrode, and the counter electrode of the glucose sensor obtained in Example 3 (2) were in contact with the hydrogel on the interstitial fluid extraction area. In this way the glucose sensor was attached to the forearm. Simultaneously with the mounting of the glucose sensor, measurement of the unprocessed current signal including the current signal attributable to the glucose was started by the glucose sensor. Measurement of the current signal by the glucose sensor was performed at two-second intervals. In this way current measurement value A1 was obtained.

(9) Blood Glucose Level Measurement

The blood glucose level of the blood was measured at the time of 3600 seconds elapsed and 23700 seconds elapsed from the start of the measurement of the unprocessed current signal including the current signal attributable to glucose in Example 3 (8), and the calibration blood glucose level $G_{3600}$ and the calibration blood glucose level $G_{23700}$ were obtained. The formula (Ic) was obtained using the current value at 3600 seconds elapsed and the current value at 23700 seconds elapsed from the start of measurement, the formula.

$$y = -0.0021 \times t + 94.661 \text{ (Ic)}$$

Note that in the formula (Ic) t represents the elapsed time from the start of the measurement of the unprocessed current signal including the current signal attributable to glucose.

The obtained formula (Ic) was used to obtain the estimated blood glucose level from the current measurement value A1t obtained in Example 3 (8).

Blood glucose levels were measured at intervals of 900 to 1800 seconds using a blood glucose self-administered measurement device from 4500 seconds elapsed after the start of measurement of an unprocessed current signal including a current signal attributable to glucose in Example 3 (8) to obtain the measured blood glucose level Gt.

(10) Estimated Blood Glucose Level Calculation

The current value Ct attributable to the test substance was calculated using the current measurement value A1t obtained in Example 3 (8) and the estimated background current value Bt at the measurement time point corresponding to the current measurement value A1t, and based on the formula (III). The measurement time point of the current signal in Example 3 (8) was divided at intervals of 5 minutes, and the average value of the current value Ct attributable to the test substance obtained within one delimited period (5 minutes) was calculated.

In order to eliminate the temporal deviation until the blood glucose level of the blood is reflected in the blood glucose value estimated from the amount of glucose in the interstitial fluid, a time correction of 152 seconds was performed on the average value of the current value Ct attributable to the test substance. Next, the estimated blood glucose level Dt was calculated based on the formula (IVd).

$$(\text{Estimated blood glucose level } Dt) = (\text{mean blood glucose level} \times i) / (-0.0021 \times t + 94.661)$$

(IVd)

Note that in the formula (IVd) the average blood glucose level is the average value of the calibration blood glucose level $G_{3600}$ and the calibration blood glucose level $G_{23700}$, where t is the elapsed time from the start of measurement of the unprocessed current signal including the current signal attributable to glucose, and i represents the current value Ct attributable to the test substance, and [- 0.0021 $\times$ t + 94.661] corresponds to the straight line represented by the formula (Ic).

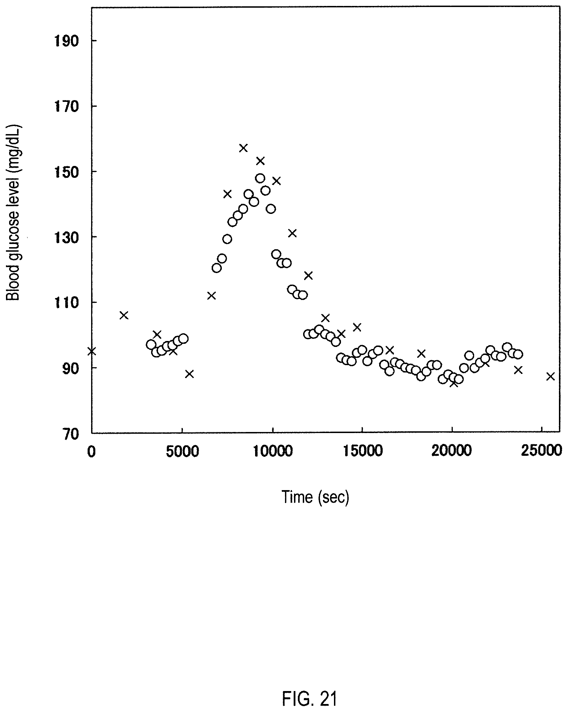

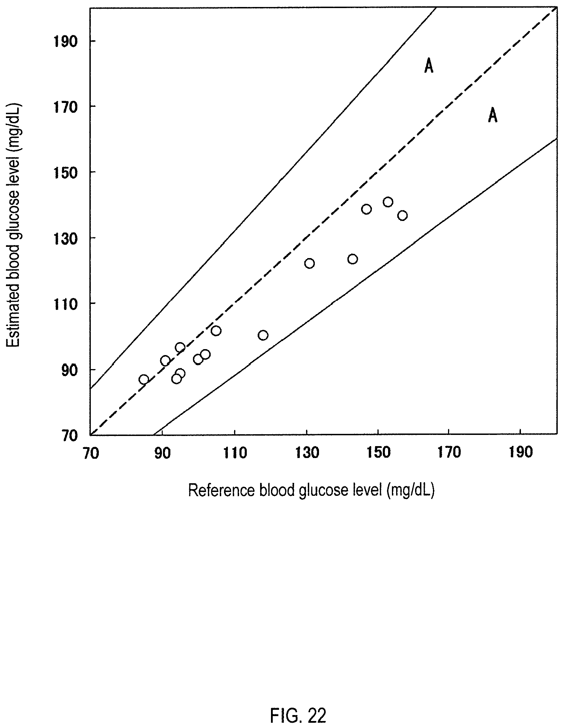

Thereafter, the changes over time in the estimated blood glucose level and the measured blood glucose level were examined. FIG. 21 shows the result of examining the change over time in blood glucose level in Example 3. In the drawing, open circles indicate change over time in the estimated blood glucose level, and crosses show change over time in the measured blood glucose level. Next, in FIG. 21, the accuracy of the estimated blood glucose level was evaluated by error grid analysis using the estimated blood glucose level and the measured blood glucose level at the same time point. The result of evaluating the estimated blood glucose level by error grid analysis in Example 3 is shown in FIG. 22. In FIG. 22, A indicates the area A in the error grid analysis.

(11) Results

From the results shown in FIG. 22, it was found that all the 14 plots of the estimated blood glucose level obtained in Example 3 were within the area A. The MARD also was 7.2%. The results suggested a highly accurate estimated blood glucose level can be obtained using the estimated background current value calculated using the background current value measured with the glucose sensor used for the measurement of glucose even when the timing of measuring the background current signal is changed from immediately before the measurement of the current signal attributable to glucose to one month before the measurement of the current signal attributable to glucose.

Performance Evaluation

Table 1 shows the ratio (hereinafter also referred to as "A area corresponding ratio") at which the estimated blood glucose levels obtained in Examples 1 to 3, and Comparative Examples 1 and 2 correspond to the A area in the error grid analysis, and MARD.

Table 1

| | Estimated background current value was used | A area corresponding ratio (%) | MARD % |
|---|---|---|---|
| Example 1 | YES | 100 | 6.3 |
| Example 2 | YES | 100 | 6.7 |
| Example 3 | YES | 100 | 7.2 |
| Comp Ex 1 | NO | 93.3 | 11.7 |
| Comp Ex 2 | NO | 89.4 | 8.1 |

As a result, the A area corresponding ratio in Examples 1 to 3 in which the estimated background current value calculated based on the measured background current value was used were all 100%. In addition, the MARD in Examples 1 to 3 were 6.3% (Example 1), 6.7% (Example 2) and 7.2% (Example 3). From these results, it was found that the accuracy of the estimated blood glucose level in Examples 1 to 3 was high.

On the other hand, the A region corresponding ratio in Comparative Examples 1 and 2 where the estimated background current value was not used were 93.3% (Comparative Example 1) and 89.4% (Comparative Example 2). In addition, the MARD in Comparative Examples 1 and 2 was 11.7% (Comparative Example 1) and 8.1 % (Comparative Example 2). From these results, it was found that the accuracy of the estimated blood glucose level in Comparative Examples 1 and 2 were lower than those in Examples 1 to 3.

It was found from Example 1 and Example 3 that an accurate estimated blood glucose level can be obtained in either case of using the estimated background current value calculated based on the background current value measured immediately before the measurement of the unprocessed current signal including the current signal attributable to glucose (Example 1), and the case of using the estimated background current value calculated based on the background current value previously measured by the glucose sensor used to measure glucose (Example 3).

Experimental Example

The working electrode, the reference electrode and the counter electrode of the glucose sensor obtained in Example 3 (2) were brought into contact with the hydrogel obtained in Example 3 (3), respectively. Next, the glucose sensor was connected to a potentiostat (trade name: ALS 832 b, manufactured by BAS, Inc.). Using a reference electrode as a reference, a voltage of 0.45 V was applied to the working electrode of the glucose sensor. A background current signal flowing between the working electrode and the counter electrode was measured for 24 hours. Note that, during the measurement of the background current signal, drying of the hydrogel was suppressed by dripping PBS onto the hydrogel.

Among the waveforms of the obtained background current signal, the background current signal corresponding to 10 minutes, 15 minutes, 20 minutes, 30 minutes, 40 minutes, 50 minutes, 60 minutes and 90 minutes from the start of measurement of the background current signal measurement were used to obtain the estimation equation shown in Table 2. By using the obtained estimation equation, the background current when a predetermined time had elapsed from the start of the measurement of the background current signal was calculated using the obtained estimation equation to obtain the estimated background current value. An error was found based on the formula (V) using the obtained estimated background current value and the measured background current value (actual measured background current value). The results are shown in Table 2 and Table 3. In the table, "measurement time" means the time of the waveform of the background current signal used in the calculation of the estimated background current value.

$$[\text{Error (\%)}] = 100 \times [\text{absolute value of (measured background current value - estimated background current value)}] / [\text{measured background current value}] \quad (V)$$

Table 2

| Measurement time (minutes) | Estimation equation | Error (%) | | |
|---|---|---|---|---|
| | | 2 hr measurement | 3 hr measurement | 4 hr measurement |
| 10 | $y=3485.9x^{-0.466}$ | 100.676 | 111.3405 | 93.01132 |
| 15 | $y=4455.7x^{-0.519}$ | 60.19827 | 65.12481 | 48.52193 |
| 20 | $y=5190.7x^{-0.549}$ | 42.97096 | 45.586 | 29.82242 |
| 30 | $y=5980.0x^{-0.576}$ | 29.59108 | 30.52462 | 15.49127 |
| 40 | $y=6726.1x^{-0.597}$ | 20.95763 | 20.79603 | 6.239419 |
| 50 | $y=7306.4x^{-0.611}$ | 16.03012 | 15.2192 | 0.927319 |
| 60 | $y=7738.4x^{-0.62}$ | 13.44947 | 12.24623 | 1.931122 |
| 90 | $y=8786.7x^{-0.64}$ | 7.852554 | 5.846845 | 8.052785 |

Table 3

| Measurement time (minutes) | Error (%) | | | | |
|---|---|---|---|---|---|
| | 21 hr measurement | 22 hr measurement | 23 hr measurement | 24 hr measurement | Total |
| 10 | 145.3509 | 183.049 | 220.0362 | 146.0658 | 102.1817 |
| 15 | 72.91278 | 98.98955 | 124.4628 | 72.19359 | 47.57708 |
| 20 | 43.80746 | 65.26409 | 86.17164 | 42.63679 | 32.35367 |
| 30 | 22.33102 | 40.40681 | 57.97995 | 20.89851 | 24.34722 |

(continued)

| Measurement time (minutes) | Error (%) | | | | |
|---|---|---|---|---|---|
| | 21 hr measurement | 22 hr measurement | 23 hr measurement | 24 hr measurement | Total |
| 40 | 8.680035 | 24.61694 | 40.08301 | 7.106613 | 21.34515 |
| 50 | 0.876636 | 15.59394 | 29.85933 | 0.769491 | 20.85662 |
| 60 | 3.432359 | 10.60996 | 24.21059 | 5.122261 | 21.84386 |
| 90 | 12.41377 | 0.229226 | 12.45341 | 14.17602 | 24.39749 |

From the results shown in Tables 2 and 3, it was found that when the measurement time is 20 to 90 minutes, and preferably 30 to 90 minutes, the error is small between the estimated background current value and the measured background current value.

EXPLANATION OF THE REFERENCE NUMERALS

10 Continuous monitoring system
20 Sensor assembly
30 Electrochemical sensor
40 Collection member
60 Terminal

**Claims**

1.   A sensor assembly comprising:

an electrochemical sensor for measuring a test substance percutaneously extracted from a living body; and a processor for determining the intensity of the background signal at an optional measurement time point of the test substance outside the predetermined time based on the intensity of the background signal measured within the predetermined time by the electrochemical sensor.

2.   The sensor assembly according to claim 1, wherein

the predetermined time is the time period from when the electrochemical sensor is energized to when the measured value by the electrochemical sensor stabilizes.

3.   The sensor assembly according to claim 1 or 2, wherein

the electrochemical sensor is configured to measure a test substance extracted into a collection member from the living body in a state in which the test substance is in contact with the collecting member; the predetermined time is the time before the test substance is extracted to the collection member after the electrochemical sensor is brought into contact with the collecting member; and the electrochemical sensor measures the intensity of the background signal within the predetermined time in a state in which the test substance is in contact with the collecting member.

4.   The sensor assembly according to any one of claims 1 or 3, wherein

the processor calculates the intensity of the background signal at the measurement time point based on the change over time in the intensity of the background signal measured within the predetermined time.

5.   The sensor assembly according to any one of claims 1 to 4, wherein

the processor calculates the intensity of the background signal at the measurement time point by applying power approximation, exponential approximation, linear approximation, or a combination thereof to the temporal change in the intensity of the background signal within the predetermined time.

6. The sensor assembly according to any one of claims 1 to 5, wherein

the processor calculates the intensity of the background signal at the measurement time point based on an equation obtained by calculating constants included in the equation by performing power approximation, exponential approximation, linear approximation or performing fitting of combinations thereof with respect to the temporal change of the intensity of the background signal within the predetermined time.

7. The sensor assembly according to any one of claims 1 to 6, further comprising:

a memory for storing the value of the intensity of the background signal at the measurement time point.

8. The sensor assembly according to any one of claims 1 to 7, wherein

the electrochemical sensor measures a test substance at each of a plurality of measurement points in time outside the predetermined time; and
the processor determines the intensity of the background signal at each of the plurality of measurement points in time based on the intensity of the background signal measured within the predetermined time.

9. The sensor assembly according to any one of claims 1 to 8, wherein

the processor obtains a value representing the intensity of the signal attributable to the test substance by subtracting the value of the intensity of the background signal at the measurement time point from the measurement value of the intensity of the unprocessed signal including the signal attributable to the test substance acquired by the electrochemical sensor at the measurement time point.

10. The sensor assembly according to any one of claims 1 to 9, wherein

the electrochemical sensor is configured to automatically obtain a value representing the intensity of the signal attributable to the test substance by measuring the intensity of the unprocessed signal including the signal attributable to the test substance and subtracting the intensity of the background signal at the measurement time point when the measurement of the intensity of the background signal and the determination of the intensity of the background signal at the measurement time point are automatically performed in a first measurement performed after the sensor assembly is first activated, and second and subsequent measurements are performed.

11. The sensor assembly according to any one of claims 1 to 10, wherein

the processor obtains a value indicating the amount of the test substance based on the intensity of the signal attributable to the test substance and the in-vivo concentration of the substance correlated with the in-vivo concentration of the test substance.

12. The sensor assembly according to claim 11, wherein

the in-vivo concentration of the substance correlated with the in-vivo concentration of the test substance is the blood concentration of the test substance.

13. The sensor assembly according to any one of claims 1 to 12 comprising one such electrochemical sensor.

14. A test substance monitoring system comprising:

a collection member capable of collecting a test substance contained in the interstitial fluid extracted from a living body; and
the sensor assembly according to any one of claims 1 to 13.

15. A monitoring method for percutaneously extracting a test substance, the method comprising:

(A1) a step of bringing a collection member into contact with an electrochemical sensor and measuring the intensity of the background signal within a predetermined time;
(A2) a step of determining the intensity of the background signal at an optional measurement time point of the test substance outside the predetermined time based on the intensity of the background signal within the predetermined time;
(A3) a step of measuring a test substance extracted into a collection member from a living body by the electrochemical sensor at the measurement time point to obtain a measurement value of the intensity of the unprocessed signal including the signal attributable to the test substance; and
(A4) a step of subtracting the value of the intensity of the background signal obtained in the step (A2) from the measured value of the intensity of the unprocessed signal measured in the step (A3) to calculate the intensity of the signal attributable to the test substance.

FIG. 1

20

21

25

22b

22a

22

22c

22d

30

23a

23

FIG. 2

FIG. 3

**60**

**20**

**40**

Terminal

121 — Communication unit

123 — Control unit

123a — Processor

123b — Memory

125 — Display unit

Sensor assembly

113 — Communication unit

115 — Control unit

115a — Processor

115b — Memory

Sensor

**30**

FIG. 4

FIG. 5

Glucose → Gluconic acid

Glucose oxidase

$2H^+ + O_2$    $H_2O_2$

$2e^-$

132

131

132

131

32

31

FIG. 6

40

42

42a

43   42b   41   44   42a   43   42b

FIG. 7

START

Measure background signal B1 — S101

Calculate estimated background signal Bt — S102

Measure unprocessed signal At including signal attributable to test substance — S103

Calculate signal Ct attributable to test substance
Ct = At - Bt — S104

Correct signal Ct attributable to test substance — S105

Output value Dt representing amount of test — S106

END

FIG. 8

FIG. 9

FIG. 10

```
                    ┌─────────────┐
                    │    START    │
                    └─────────────┘
                           │
                           ▼
   ┌──────────────────────────────────────────────┐
   │  Measure unprocessed signal At including signal│──── S201
   │       attributable to test substance          │
   └──────────────────────────────────────────────┘
                           │
                           ▼
   ┌──────────────────────────────────────────────┐
   │       Call estimated background signal Bt      │──── S202
   └──────────────────────────────────────────────┘
                           │
                           ▼
   ┌──────────────────────────────────────────────┐
   │ Calculate signal Ct attributable to test substance│──── S203
   │                  Ct = At - Bt                  │
   └──────────────────────────────────────────────┘
                           │
                           ▼
   ┌──────────────────────────────────────────────┐
   │  Correct signal Ct attributable to test substance│──── S204
   └──────────────────────────────────────────────┘
                           │
                           ▼
   ┌──────────────────────────────────────────────┐
   │  Output value Dt representing amount of test   │──── S205
   │                  substance                     │
   └──────────────────────────────────────────────┘
                           │
                           ▼
                    ┌─────────────┐
                    │     END     │
                    └─────────────┘
```

FIG. 11

FIG. 12

FIG. 13

FIG. 14

EP 3 289 976 A1

FIG. 15

FIG. 16

FIG. 17

FIG. 18

FIG. 19

FIG. 20

FIG. 21

FIG. 22

FIG. 23

FIG. 24

Europäisches
Patentamt

European
Patent Office

Office européen
des brevets

# EUROPEAN SEARCH REPORT

Application Number

EP 17 18 8116

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2005/215872 A1 (BERNER BRET [US] ET AL) 29 September 2005 (2005-09-29) * paragraphs [0096], [0128] * | 1-15 | INV. A61B5/145 A61B5/00 A61B5/1495 |
| X | US 2002/002328 A1 (TAMADA JANET [US]) 3 January 2002 (2002-01-03) * paragraphs [0073], [0082] * | 1-15 | |
| X | US 2001/016682 A1 (BERNER BRET [US] ET AL) 23 August 2001 (2001-08-23) * paragraphs [0087], [0106], [0127], [0128] * | 1-15 | |
| X | US 2003/235817 A1 (BARTKOWIAK MIROSLAW [US] ET AL) 25 December 2003 (2003-12-25) * paragraphs [0112], [0119], [0125], [0176] - [0231] * | 1-15 | |

TECHNICAL FIELDS
SEARCHED      (IPC)

A61B

The present search report has been drawn up for all claims

| Place of search | Date of completion of the search | Examiner |
|---|---|---|
| Berlin | 8 January 2018 | Trachterna, Morten |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

**ANNEX TO THE EUROPEAN SEARCH REPORT
ON EUROPEAN PATENT APPLICATION NO.** EP 17 18 8116

This annex lists the patent family members relating to the patent documents cited in the above-mentioned European search report.
The members are as contained in the European Patent Office EDP file on
The European Patent Office is in no way liable for these particulars which are merely given for the purpose of information.

08-01-2018

| Patent document cited in search report | | Publication date | Patent family member(s) | | Publication date |
|---|---|---|---|---|---|
| US 2005215872 | A1 | 29-09-2005 | AT | 245937 T | 15-08-2003 |
| | | | CA | 2332112 A1 | 18-11-1999 |
| | | | DE | 69910003 D1 | 04-09-2003 |
| | | | DE | 69910003 T2 | 22-04-2004 |
| | | | DK | 1077634 T3 | 24-11-2003 |
| | | | EP | 1077634 A1 | 28-02-2001 |
| | | | ES | 2205818 T3 | 01-05-2004 |
| | | | JP | 3600158 B2 | 08-12-2004 |
| | | | JP | 2002514453 A | 21-05-2002 |
| | | | PT | 1077634 E | 31-12-2003 |
| | | | US | 6144869 A | 07-11-2000 |
| | | | US | 6356776 B1 | 12-03-2002 |
| | | | US | 2002091312 A1 | 11-07-2002 |
| | | | US | 2003195403 A1 | 16-10-2003 |
| | | | US | 2005215872 A1 | 29-09-2005 |
| | | | US | 2007142721 A1 | 21-06-2007 |
| | | | WO | 9958051 A1 | 18-11-1999 |
| US 2002002328 | A1 | 03-01-2002 | AT | 255936 T | 15-12-2003 |
| | | | AU | 2902695 A | 19-01-1996 |
| | | | CA | 2193884 A1 | 04-01-1996 |
| | | | DE | 69532282 D1 | 22-01-2004 |
| | | | DE | 69532282 T2 | 09-12-2004 |
| | | | DK | 0766577 T3 | 22-03-2004 |
| | | | EP | 0766577 A1 | 09-04-1997 |
| | | | ES | 2211909 T3 | 16-07-2004 |
| | | | JP | 3350053 B2 | 25-11-2002 |
| | | | JP | H10505761 A | 09-06-1998 |
| | | | PT | 766577 E | 31-03-2004 |
| | | | US | 5771890 A | 30-06-1998 |
| | | | US | 6023629 A | 08-02-2000 |
| | | | US | 2001020124 A1 | 06-09-2001 |
| | | | US | 2002002328 A1 | 03-01-2002 |
| | | | WO | 9600109 A1 | 04-01-1996 |
| US 2001016682 | A1 | 23-08-2001 | AT | 258028 T | 15-02-2004 |
| | | | CA | 2311487 A1 | 18-11-1999 |
| | | | DE | 69914319 D1 | 26-02-2004 |
| | | | DE | 69914319 T2 | 18-11-2004 |
| | | | DK | 1077636 T3 | 24-05-2004 |
| | | | EP | 1077636 A1 | 28-02-2001 |
| | | | ES | 2213369 T3 | 16-08-2004 |
| | | | JP | 4545398 B2 | 15-09-2010 |
| | | | JP | 2002514452 A | 21-05-2002 |
| | | | JP | 2004000655 A | 08-01-2004 |
| | | | PT | 1077636 E | 30-06-2004 |

For more details about this annex : see Official Journal of the European Patent Office, No. 12/82

EPO FORM P0459

page 1 of 2

## ANNEX TO THE EUROPEAN SEARCH REPORT
## ON EUROPEAN PATENT APPLICATION NO.

EP 17 18 8116

This annex lists the patent family members relating to the patent documents cited in the above-mentioned European search report.
The members are as contained in the European Patent Office EDP file on
The European Patent Office is in no way liable for these particulars which are merely given for the purpose of information.

08-01-2018

| Patent document cited in search report | Publication date | Patent family member(s) | | Publication date |
|---|---|---|---|---|
| | | US | 6233471 B1 | 15-05-2001 |
| | | US | 2001016682 A1 | 23-08-2001 |
| | | US | 2003153820 A1 | 14-08-2003 |
| | | US | 2003153821 A1 | 14-08-2003 |
| | | US | 2007038053 A1 | 15-02-2007 |
| | | WO | 9958050 A1 | 18-11-1999 |
| US 2003235817 A1 | 25-12-2003 | AT | 482649 T | 15-10-2010 |
| | | AT | 507766 T | 15-05-2011 |
| | | CA | 2480550 A1 | 09-10-2003 |
| | | CA | 2738579 A1 | 09-10-2003 |
| | | EP | 1489961 A2 | 29-12-2004 |
| | | EP | 1702561 A2 | 20-09-2006 |
| | | ES | 2357318 T3 | 25-04-2011 |
| | | HK | 1074371 A1 | 08-04-2011 |
| | | HK | 1090532 A1 | 25-11-2011 |
| | | JP | 4083689 B2 | 30-04-2008 |
| | | JP | 4574646 B2 | 04-11-2010 |
| | | JP | 4574647 B2 | 04-11-2010 |
| | | JP | 4588742 B2 | 01-12-2010 |
| | | JP | 4615547 B2 | 19-01-2011 |
| | | JP | 2005520662 A | 14-07-2005 |
| | | JP | 2007252954 A | 04-10-2007 |
| | | JP | 2007252955 A | 04-10-2007 |
| | | JP | 2007252956 A | 04-10-2007 |
| | | JP | 2007256296 A | 04-10-2007 |
| | | US | 2003235817 A1 | 25-12-2003 |
| | | US | 2006063218 A1 | 23-03-2006 |
| | | US | 2006074564 A1 | 06-04-2006 |
| | | US | 2006085137 A1 | 20-04-2006 |
| | | WO | 03082098 A2 | 09-10-2003 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 4545398 B